# EUROPEAN PATENT SPECIFICATION

(11) **EP 4 267 031 B1**
(45) Date of publication and mention of the grant of the patent: **16.09.2026**
(21) Application number: 20855887.4
(22) Date of filing: 26.12.2020
(51) Int. Cl.: A61B 34/30

(54) **CATHETER ROBOT MODULE FOR TRANSLATION AND ROTATION OF A FLEXIBLE ELONGATED MEDICAL ELEMENT**
KATHETERROBOTERMODUL ZUR TRANSLATION UND ROTATION EINES FLEXIBLEN LÄNGLICHEN MEDIZINISCHEN ELEMENTS
MODULE DE ROBOT DE CATHÉTER PERMETTANT UN MOUVEMENT DE TRANSLATION ET DE ROTATION D'UN ÉLÉMENT MÉDICAL ALLONGÉ SOUPLE

(43) Date of publication of application: 01.11.2023
(73) Proprietor: Robocath, 76000 Rouen (FR)
(72) Inventor: FOURNIER, Bruno, 41100 Saint Ouen (FR)
(74) Representative: Plasseraud IP
(86) International application number: PCT/IB2020/001116
(87) International publication number: WO 2022/136894

(56) References cited:
- WO-A1-2018/189473
- US-A1- 2014 277 334
- US-A1- 2015 001 968
- US-A1- 2015 297 864
- US-A1- 2017 151 024
- US-A1- 2019 351 187

## Description

### FIELD OF THE INVENTION

The invention relates to the technical field of catheter robot modules for translation and rotation of a flexible elongated medical element. This flexible elongated medical element can be guide of a catheter and/or a catheter, and/or a catheter guide. Usually, these elements are disposed so that, at least partly, i.e. on part of their respective length, the catheter guide surrounds the catheter which itself surrounds the guide of a catheter.

### BACKGROUND OF THE INVENTION

According to a prior art, described in EP 15733825, and belonging to the same assignee Robocath, a catheter robot module is described which includes a pair of movable pads configured to clamp and unclamp a flexible elongated medical element. This pair of movable pads is also disposed so as to be able to impart to this flexible elongated medical element, either a translation move and/or a rotation move. This pair of movable pads can translate the flexible elongated medical element like fingers of two hands would pull this flexible elongated medical element forward. This pair of movable pads can rotate the flexible elongated medical element like fingers of a hand would make this flexible elongated medical element roll between those fingers.

Unlike the manual move of hand fingers, which is first rather slow and which is second assisted by the practitioner's brain whose hands manipulates the flexible elongated medical element, the catheter robot module becomes more and more interesting when the translation and rotation moves of the flexible elongated medical element can be made quicker and quicker. However, the translation speed and rotation speed, as well as the translation speed variations and the rotation speed variations, become rapidly limited, because global synchronization, between on one side translation and rotation moves of each pair of movable pads, and between on the other side both pairs of movable pads, each pair performing translation and rotation, soon becomes hard to manage, when the translation and rotation speeds increase, and also when the rapidity of variations allowed for these translation and rotation speeds increase. Increasing speeds and speed variations, not only improves catheter robot module efficiency to provide the physician with agility in difficult situations such as crossing a lesion or selecting an arterial side branch, but also increases its security allowing for quick reactions in case of incident or in case of danger risk.

No prior art has tried and tackled these synchronization problems when speeds and speed variations increase. When looking into them, these synchronization problems appear to be at first sight complex and intricate. Other relevant prior art can be found in US 2014/277334 A1, WO 2018/189473 A1, US 2015/001968 A1, US 2019/351187 A1 and US 2015/297864 A1.

### SUMMARY OF THE INVENTION

The invention is defined by the appended claims.

The object of the present invention is to alleviate at least partly the above-mentioned drawbacks.

More particularly, the invention technical contribution is of two kinds:
➢ First, it has split and ordered this complex global synchronization task into at least two more simple specific synchronization tasks, which are:
   ∘ Control of opposition phase between the two pairs of movable pads,
   ∘ And management of clamping conflict between the two pairs of movable pads,
➢ Second, it has provided for each one of these two specific synchronization tasks:
   ∘ Not only an efficient technical solution,
   ∘But also a technical solution which is compatible with the technical solution of the other specific synchronization task, and can be synchronized with it,
      ▪ Thereby allowing for use of both these technical solutions to provide a global answer, to the complex global synchronization task existing between both pairs of movable pads.

So, technical contribution of the invention includes:
➢ Spitting global complex synchronization task between both pairs of movable pads into two specific simpler synchronization tasks which are, first controlling and keeping phase opposition between both pairs of movable pads, and second always keeping at least one pair of movable pads clamped on the flexible elongated medical element,
➢ Bringing technical solutions respectively to these two specific simpler synchronization tasks, which:
   ∘ Not only solve the problems of these two specific simpler synchronization tasks,
   ∘ But also solve these problems in a compatible and even more easily synchronized way, so that the problems of both these two specific simpler synchronization tasks can be solved simultaneously, while keeping the global catheter robot module in a reasonable complexity and cost.

However, the present invention mainly focuses on providing a specific technical solution to the specific simpler synchronization task existing between both pairs of movable pads, which deals with always keeping at least one pair of movable pads clamped on the flexible elongated medical element, what will be useful for improving the process and going toward a rather rapid, fluid and secure control of the moving flexible elongated medical element.

The main technical contribution of the invention deals with:
➢ A variation of a travel extension of a forth translation in a first translation cycle for at least one of said pairs, and/or variation of a travel extension and/or a duration of a forth translation in a second rotation cycle for at least one of the two pairs of movable pads,
   ∘ So as to always keep at least one pair of movable pads clamped on the flexible elongated medical element.

In a preferred embodiment, so as to further improve the process and to get at a rapid, fluid and secure control of the moving flexible elongated medical element, there is a supplementary technical contribution of the invention which deals with:
➢ An additional variation of a duration of a translating back phase in a first translation cycle for at least one of the two pairs of movable pads,
   ∘ So as to control and keep phase opposition between both pairs of movable pads.

This object is achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to, separately or in combination:
      ▪ perform a translation of said flexible elongated medical element longitudinally with respect to said casing, by a first translation cycle:
         - clamping said flexible elongated medical element between said pads,
         - translating forth said pads synchronously longitudinally in the same direction with respect to said casing, with respect to a user set longitudinal translation direction,
         - unclamping said flexible elongated medical element,
         - translating back said pads synchronously longitudinally in the same reverse direction with respect to said casing, with respect to said user set longitudinal translation direction,
      ▪ perform a rotation of said flexible elongated medical element around longitudinal axis with respect to said casing, by a second rotation cycle:
         - clamping said flexible elongated medical element between said pads,
         - performing a relative forth translation of said pads transversely in opposite directions with respect to said casing, with respect to a transversal translation direction corresponding to a set rotation direction,
         - unclamping said flexible elongated medical element,
         - performing a relative back translation of said pads transversely in opposite reverse directions with respect to said casing, with respect to said transversal translation direction corresponding to said set rotation direction,
➢ a driver of said pairs of movable pads implemented so that:
   ∘ in at least one mode where, in combination, said translation of said flexible elongated medical element is alternatively performed by said pairs of movable pads, both said pairs working in phase opposition, and said rotation of said flexible elongated medical element is performed by at least one of said pairs of movable pads,
      ▪ conflict of synchronization between said translation and said rotation is managed at least:
         - by varying travel extension of said forth translation in said first translation cycle for at least one of said pairs, and/or by varying travel extension and/or duration of said forth translation in said second rotation cycle for at least one of said pairs,
            ∘ so as to always keep at least one pair of movable pads clamped on said flexible elongated medical element, during the whole duration of said translation of said flexible elongated medical element in said first translation cycle as well as during the whole duration of said rotation of said flexible elongated medical element in said second rotation cycle.

Preferably, said driver of said pairs of movable pads is also implemented so that:
∘ in one or several or all modes where said translation of said flexible elongated medical element is performed:
   ▪ said translation of said flexible elongated medical element is alternatively performed by said pairs of movable pads, both said pairs working in phase opposition, said phase opposition being controlled at least:
      - by varying duration of said translating back in said first translation cycle for at least one of said pairs,
         ∘ so as to control and keep said phase opposition between both said pairs.

This object can be achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to, separately or in combination:
      ▪ perform a translation of said flexible elongated medical element longitudinally with respect to said casing, by a first translation cycle:
         - clamping said flexible elongated medical element between said pads,
         - translating forth said pads synchronously longitudinally in the same direction with respect to said casing, with respect to a user set longitudinal translation direction,
         - unclamping said flexible elongated medical element,
         - translating back said pads synchronously longitudinally in the same reverse direction with respect to said casing, with respect to said user set longitudinal translation direction,
         - like fingers of a hand pulling said flexible elongated medical element forward,
      ▪ perform a rotation of said flexible elongated medical element around longitudinal axis with respect to said casing, by a second rotation cycle:
         - clamping said flexible elongated medical element between said pads,
         - performing a relative forth translation of said pads transversely in opposite directions with respect to said casing, with respect to a transversal translation direction corresponding to a set rotation direction,
         - unclamping said flexible elongated medical element,
         - performing a relative back translation of said pads transversely in opposite reverse directions with respect to said casing, with respect to said transversal translation direction corresponding to said set rotation direction,
         - like fingers of a hand making said flexible elongated medical element rolling between them,
➢ a driver of said pairs of movable pads implemented so that:
   ∘ in at least one mode where, in combination, said translation of said flexible elongated medical element is alternatively performed by said pairs of movable pads, both said pairs working in phase opposition, and said rotation of said flexible elongated medical element is performed by at least one of said pairs of movable pads,
      ▪ conflict of synchronization between said translation and said rotation is managed at least:
         - by varying travel extension of said forth translation in said first translation cycle for at least one of said pairs, and/or by varying travel extension and/or duration of said forth translation in said second rotation cycle for at least one of said pairs,
            ∘ so as to always keep at least one pair of movable pads clamped on said flexible elongated medical element, during the whole duration of said translation of said flexible elongated medical element in said first translation cycle as well as during the whole duration of said rotation of said flexible elongated medical element in said second rotation cycle.

Preferably, said driver of said pairs of movable pads is also implemented so that:
∘ in one or several or all modes where said translation of said flexible elongated medical element is performed:
   ▪ said translation of said flexible elongated medical element is alternatively performed by said pairs of movable pads, both said pairs working in phase opposition, said phase opposition being controlled at least:
      - by varying duration of said translating back in said first translation cycle for at least one of said pairs,
         ∘ so as to control and keep said phase opposition between both said pairs.

This object is also achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to, separately or in combination:
      ▪ perform a translation of said flexible elongated medical element longitudinally with respect to said casing, by a first translation cycle which clamps, translates forth, unclamps, and translates back, depending on a user set longitudinal translation direction,
      ▪ perform a rotation of said flexible elongated medical element around longitudinal axis with respect to said casing, by a second rotation cycle which clamps, performs a relative forth translation of said pads in opposite directions, unclamps, performs a relative back translation of said pads in opposite directions, depending on a set rotation direction,
➢ a driver of said pairs of movable pads implemented so that:
   ∘ conflict of synchronization, between said translation alternatively performed by said pairs of movable pads working in phase opposition and said rotation, when in combination, is managed at least:
      ▪ by varying travel extension and/or duration of said forth translation, in said first translation cycle and/or in said second rotation cycle, for at least one of said pairs,
         - so as to always keep at least one pair of movable pads clamped on said flexible elongated medical element, during said translation and during said rotation.

Preferably, said driver of said pairs of movable pads is also implemented so that:
∘ said translation of said flexible elongated medical element is alternatively performed by said pairs of movable pads, both said pairs working in phase opposition, said phase opposition being controlled at least:
   - by varying duration of said translating back in said first translation cycle for at least one of said pairs,
      ∘ so as to control and keep said phase opposition between both said pairs.

This object can be also achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to, separately or in combination:
      ▪ perform a translation of said flexible elongated medical element longitudinally with respect to said casing, like fingers of a hand pulling said flexible elongated medical element forward, by a first translation cycle which clamps, translates forth, unclamps, and translates back, depending on a user set longitudinal translation direction,
      ▪ perform a rotation of said flexible elongated medical element around longitudinal axis with respect to said casing, like fingers of a hand making said flexible elongated medical element rolling between them, by a second rotation cycle which clamps, performs a relative forth translation of said pads in opposite directions, unclamps, performs a relative back translation of said pads in opposite directions, depending on a set rotation direction,
➢ a driver of said pairs of movable pads implemented so that:
   ∘ conflict of synchronization, between said translation alternatively performed by said pairs of movable pads working in phase opposition and said rotation, when in combination, is managed at least:
      ▪ by varying travel extension and/or duration of said forth translation, in said first translation cycle and/or in said second rotation cycle, for at least one of said pairs,
         - so as to always keep at least one pair of movable pads clamped on said flexible elongated medical element, during said translation and during said rotation.

Preferably, said driver of said pairs of movable pads is also implemented so that:
∘said translation of said flexible elongated medical element is alternatively performed by said pairs of movable pads, both said pairs working in phase opposition, said phase opposition being controlled at least:
   - by varying duration of said translating back in said first translation cycle for at least one of said pairs,
      ∘ so as to control and keep said phase opposition between both said pairs.

Said rotation of said flexible elongated medical element may be performed by only one of said pairs of movable pads.

Preferably, said rotation of said flexible elongated medical element is alternatively performed by said pairs of movable pads. Hence, global move of the flexible elongated medical element can be made more fluid and rapid, to the cost of an additional complexity, the ability of a second pair of movable pads to perform rotation of the flexible elongated element.

In this later case, said rotation of said flexible elongated medical element is preferably alternatively performed by said pairs of movable pads, both said pairs working in phase opposition, said phase opposition being controlled, at least by varying duration of said translating back in said second rotation cycle for at least one of said pairs, so as to control and keep said phase opposition between both said pairs.

In the case where said rotation of said flexible elongated medical element is alternatively performed by said pairs of movable pads, this object is still achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to separately or in combination:
      ▪ perform a translation of said flexible elongated medical element longitudinally with respect to said casing, by a first translation cycle:
         - clamping said flexible elongated medical element between said pads,
         - translating forth said pads synchronously longitudinally in the same direction with respect to said casing, with respect to a user set longitudinal translation direction,
         - unclamping said flexible elongated medical element,
         - translating back said pads synchronously longitudinally in the same reverse direction with respect to said casing, with respect to said user set longitudinal translation direction,
      ▪ perform a rotation of said flexible elongated medical element around longitudinal axis with respect to said casing, by a second rotation cycle:
         - clamping said flexible elongated medical element between said pads,
         - performing a relative forth translation of said pads transversely in opposite directions with respect to said casing, with respect to a transversal translation direction corresponding to a set rotation direction,
         - unclamping said flexible elongated medical element,
         - performing a relative back translation of said pads transversely in opposite reverse directions with respect to said casing, with respect to said transversal translation direction corresponding to said set rotation direction,
➢ a driver of said pairs of movable pads implemented so that:
   ∘ said translation of said flexible elongated medical element being alternatively performed by said pairs of movable pads, both said pairs working in phase opposition,
   ∘ said rotation of said flexible elongated medical element being alternatively performed by said pairs of movable pads,
   ∘ conflict of synchronization between said translation and said rotation being managed,
   ∘ at least:
      ▪ by varying travel extension and/or duration of said forth translation, in said first translation cycle and/or in said second rotation cycle, for both of said pairs,
         - so as to always keep at least one pair of movable pads clamped on said flexible elongated medical element, during the whole duration of said translation of said flexible elongated medical element in said first translation cycle as well as during the whole duration of said rotation of said flexible elongated medical element in said second rotation cycle.

Preferably, said driver of said pairs of movable pads is also implemented so that:
∘ said phase opposition is controlled, at least:
   ▪ by varying duration of said translating back, in said first translation cycle and in said second rotation cycle, for at least one of said pairs,
      - so as to control and keep said phase opposition between both said pairs.

In the case where said rotation of said flexible elongated medical element is alternatively performed by said pairs of movable pads, this object can be still achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to separately or in combination:
      ▪ perform a translation of said flexible elongated medical element longitudinally with respect to said casing, by a first translation cycle:
         - clamping said flexible elongated medical element between said pads,
         - translating forth said pads synchronously longitudinally in the same direction with respect to said casing, with respect to a user set longitudinal translation direction,
         - unclamping said flexible elongated medical element,
         - translating back said pads synchronously longitudinally in the same reverse direction with respect to said casing, with respect to said user set longitudinal translation direction,
         - like fingers of a hand pulling said flexible elongated medical element forward,
      ▪ perform a rotation of said flexible elongated medical element around longitudinal axis with respect to said casing, by a second rotation cycle:
         - clamping said flexible elongated medical element between said pads,
         - performing a relative forth translation of said pads transversely in opposite directions with respect to said casing, with respect to a transversal translation direction corresponding to a set rotation direction,
         - unclamping said flexible elongated medical element,
         - performing a relative back translation of said pads transversely in opposite reverse directions with respect to said casing, with respect to said transversal translation direction corresponding to said set rotation direction,
         - like fingers of a hand making said flexible elongated medical element rolling between them,
➢ a driver of said pairs of movable pads implemented so that:
   ∘ said translation of said flexible elongated medical element being alternatively performed by said pairs of movable pads, both said pairs working in phase opposition,
   ∘ said rotation of said flexible elongated medical element being alternatively performed by said pairs of movable pads,
   ∘ conflict of synchronization between said translation and said rotation being managed,
   ∘ at least:
      ▪ by varying travel extension and/or duration of said forth translation, in said first translation cycle and/or in said second rotation cycle, for both of said pairs,
         - so as to always keep at least one pair of movable pads clamped on said flexible elongated medical element, during the whole duration of said translation of said flexible elongated medical element in said first translation cycle as well as during the whole duration of said rotation of said flexible elongated medical element in said second rotation cycle.

Preferably, said driver of said pairs of movable pads is also implemented so that:
∘ said phase opposition is controlled, at least:
   ▪ by varying duration of said translating back, in said first translation cycle and in said second rotation cycle, for at least one of said pairs,
      - so as to control and keep said phase opposition between both said pairs.

In the case where said rotation of said flexible elongated medical element is alternatively performed by said pairs of movable pads, this object is still achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to separately or in combination:
      ▪ perform a translation of said flexible elongated medical element longitudinally with respect to said casing, by a first translation cycle which clamps, translates forth, unclamps, and translates back, depending on a user set longitudinal translation direction,
      ▪ perform a rotation of said flexible elongated medical element around longitudinal axis with respect to said casing, by a second rotation cycle which clamps, performs a relative forth translation of said pads in opposite directions, unclamps, performs a relative back translation of said pads in opposite directions, depending on a set rotation direction,
➢ a driver of said pairs of movable pads implemented so that:
   ∘ said translation of said flexible elongated medical element being alternatively performed by said pairs of movable pads, both said pairs working in phase opposition,
   ∘ said rotation of said flexible elongated medical element being alternatively performed by said pairs of movable pads,
   ∘ conflict of synchronization between said translation and said rotation being managed, at least:
      ▪ by varying travel extension and/or duration of said forth translation, in said first translation cycle and/or in said second rotation cycle, for both of said pairs,
         - so as to always keep at least one pair of movable pads clamped on said flexible elongated medical element, during said translation and during said rotation.

Preferably, said driver of said pairs of movable pads is also implemented so that:
∘ said phase opposition is controlled, at least:
   ▪ by varying duration of said translating back, in said first translation cycle and in said second rotation cycle, for at least one of said pairs,
      - so as to control and keep said phase opposition between both said pairs.

In the case where said rotation of said flexible elongated medical element is alternatively performed by said pairs of movable pads, this object can be still achieved with a catheter robot module for translation and rotation of a flexible elongated medical element, comprising:
➢ a casing,
➢ two pairs of movable pads:
   ∘ said pads of a same pair at least partly facing each other,
   ∘ each pair of movable pads being adapted to separately or in combination:
      ▪ perform a translation of said flexible elongated medical element longitudinally with respect to said casing, like fingers of a hand pulling said flexible elongated medical element forward, by a first translation cycle which clamps, translates forth, unclamps, and translates back, depending on a user set longitudinal translation direction,
      ▪ perform a rotation of said flexible elongated medical element around longitudinal axis with respect to said casing, like fingers of a hand making said flexible elongated medical element rolling between them, by a second rotation cycle which clamps, performs a relative forth translation of said pads in opposite directions, unclamps, performs a relative back translation of said pads in opposite directions, depending on a set rotation direction,
➢ a driver of said pairs of movable pads implemented so that:
   ∘ said translation of said flexible elongated medical element being alternatively performed by said pairs of movable pads, both said pairs working in phase opposition,
   ∘ said rotation of said flexible elongated medical element being alternatively performed by said pairs of movable pads,
   ∘ conflict of synchronization between said translation and said rotation being managed, at least:
      ▪ by varying travel extension and/or duration of said forth translation, in said first translation cycle and/or in said second rotation cycle, for both of said pairs,
         - so as to always keep at least one pair of movable pads clamped on said flexible elongated medical element, during said translation and during said rotation.

Preferably, said driver of said pairs of movable pads is also implemented so that:
∘ said phase opposition is controlled, at least:
   ▪ by varying duration of said translating back, in said first translation cycle and in said second rotation cycle, for at least one of said pairs,
      - so as to control and keep said phase opposition between both said pairs.

Preferred embodiments comprise one or more of the following features, which can be taken separately or together, either in partial combination or in full combination.

Preferably, said driver of said pairs of movable pads is implemented so that said translation of said flexible elongated medical element is alternatively performed by said pairs of movable pads, both pairs working in phase opposition, said phase opposition being controlled mainly or only, by varying duration of said translating back, in said first translation cycle for at least one of said pairs, so as to control and keep said phase opposition between both said pairs.

Hence, variation of this key parameter, duration of said translating back, in said first translation cycle for at least one of said pairs, may be sufficient to control and keep said phase opposition between both said pairs.

Preferably, said driver of said pairs of movable pads is implemented so that said rotation of said flexible elongated medical element is alternatively performed by said pairs of movable pads, conflict of synchronization between said translation and said rotation is managed mainly or only, by varying travel extension of said forth translation, in said first translation cycle for at least one of said pairs, so as to always keep at least one pair of movable pads clamped on said flexible elongated medical element, during the whole duration of said translation of said flexible elongated medical element in said first cycle as well as during the whole duration of said rotation of said flexible elongated medical element in said second cycle.

Hence, variation of this key parameter, travel extension of said forth translation, in said first translation cycle for at least one of said pairs, may be sufficient to always keep at least one pair of movable pads clamped on said flexible elongated medical element.

Preferably, said forth translation duration is always longer than said back translation duration.

Hence, the catheter robot module is more efficient since main part of the time is dedicated to move the flexible elongated medical element in the targeted direction rather than to bring back the pairs of movable pads in the reverse direction for next pull of this flexible elongated medical element.

Preferably, said varying travel extension of said forth translation in said first translation cycle for one of said pairs is performed by extending a predetermined standard forth translation travel range, reaching a value ranging from said predetermined standard forth translation travel range to a predetermined maximum forth translation travel range.

Hence, this is a simple and efficient way to provide for a delay before unclamping, by providing for an extra travel in such a way that the perturbation before getting back at standard path with synchronized moves between the two pairs of movable pads is minimized.

Preferably, said predetermined maximum forth translation travel range is comprised between 110% and 150% of said predetermined standard forth translation travel range, preferably between 120% and 140% of said predetermined standard forth translation travel range.

Hence, there is a good compromise between:
➢ the global efficiency in most cases where only the standard travel is needed,
➢ and the high security in few cases where a notable extra travel may be needed.

Preferably, said predetermined maximum forth translation travel range is split in two equal parts respectively at both ends of said predetermined standard forth translation travel range.

Hence, both positive and negative targeted speed values chosen by user can be both efficiently managed.

Preferably, there is some temporary overlapping between said flexible elongated medical element clamping by one of said pairs of movables pads and said flexible elongated medical element clamping by the other one of said pairs of movables pads, said temporary overlapping lasting preferably between 10% and 95% of the whole duration of said translation of said flexible elongated medical element.

Hence, the security is improved, by increasing the time when both pairs of movable clamps are simultaneously clamped.

Preferably, said flexible elongated medical element unclamping is performed simultaneously to a portion of said forth translation travel extension, during the second half of said forth translation travel extension, said portion ranging preferably from 5% to 20% of the full extent of said forth translation travel extension.

Hence, the fluidity of the flexible elongated medical element clamping and bringing back pairs of movable pads before next flexible elongated medical element pulling is improved.

Preferably, said flexible elongated medical element clamping is performed simultaneously to a portion of said forth translation travel extension, during the first half of said forth translation travel extension, said portion ranging preferably from 5% to 20% of the full extent of said forth translation travel extension.

Hence, the fluidity of the flexible elongated medical element clamping and pulling is improved.

Preferably, said flexible elongated medical element clamping starts after the end of said back translation travel extension and after the beginning of next said forth translation travel extension.

Hence, the fluidity of the flexible elongated medical element clamping and pulling is improved.

Preferably, said varying duration of said translating back in said first translation cycle for one of said pairs, so as to control and keep said phase opposition between both said pairs, is performed by reducing or extending duration (and thus speed) with respect to a standard back translation duration.

Hence, this is a simple and efficient way to provide for a resynchronization, by providing for an extra duration range, either to increase or to decrease a standard duration, in such a way that the perturbation before getting back at standard path with synchronized moves between the two pairs of movable pads is minimized.

Preferably, said varying duration of said translating back in said first translation cycle for one of said pairs, so as to control and keep said phase opposition between both said pairs, is performed by reducing or extending duration with respect to a standard back translation duration less than requested for optimal phase opposition controlling and keeping so as to improve stability to the cost of higher number of cycles to get back at phase opposition target, a factor α of correction attenuation comprised between 0 and 1 being applied.

Hence, by reducing or extending duration by an amount less than what would be needed for a complete and full phase opposition control, a gain in stability can be obtained, even if such stability is not regained immediately from last instability.

Preferably, said factor α of correction attenuation is comprised between 0.3 and 0.7, and is preferably about 0.5.

Hence, the compromise between stability level and rapidity to regain stability after last instability can be optimized.

Preferably, said standard back translation duration is a decreasing function of a user command speed target value(s), for either translation and/or rotation, preferably minimum of both speed target values, when applicable, becoming selected user command speed target value.

Hence, the rapidity to correct deviations from standard working is better adapted to the translation and rotation speeds requested by the user.

Preferably, said decreasing function presents a central curved part which presents a concavity toward top and which is located between two horizontal parts.

Hence, the two horizontal parts allow for fluid and correct working of the correction. Indeed, the upper horizontal part avoids too long unclamping period which would increase the number of clamping conflicts happening. Indeed, the lower horizontal part avoids too much demand on the response time of the actuators which are limited in speed.

Preferably, said central curved part is inversely proportional to said selected user command speed target value, whereas said horizontal parts are constant with respect to said selected user command speed target value.

Hence, the rapidity to correct deviations from standard working is better adapted to the translation and rotation speeds requested by the user.

Preferably, said user set longitudinal translation direction can be varied continuously by said user, and/or said user set rotation direction can be varied continuously by said user.

Hence, the catheter robot module is more flexible and thereby more useful to the user.

Preferably, said translation of said flexible elongated medical element longitudinally for first of said pairs of pads is performed using several steps controlled by a first finite state machine, said rotation of said flexible elongated medical element around longitudinal axis with respect to said casing for first of said pairs of pads is performed using several steps controlled by a second finite state machine, said translation of said flexible elongated medical element longitudinally for second of said pairs of pads is performed using several steps controlled by a third finite state machine, said rotation of said flexible elongated medical element around longitudinal axis with respect to said casing for second of said pairs of pads is performed using several steps controlled by a fourth finite state machine.

Hence, the compromise between complexity and efficiency is improved.

Preferably, each of said finite state machines determines for a transition period between said forth translation and said back translation to go progressively from said forth translation to said back translation: start of said transition period, duration of said transition period, end of said transition period.

Hence, the global process moving the flexible elongated medical element is more fluid.

Preferably, each of said finite state machines has all its state variables updated periodically with a period which is less than 5ms, preferably comprised between 0.5ms and 2ms, more preferably about 1ms.

Hence, the global process moving the flexible elongated medical element is also more reactive, while not losing its fluidity.

Further features and advantages of the invention will appear from the following description of embodiments of the invention, given as non-limiting examples, with reference to the accompanying drawings listed hereunder.

### BRIEF DESCRIPTION OF THE DRAWINGS

Fig. 1 shows schematically two pairs of two movable pads each, within an example of a catheter robot module according to the invention.
Fig. 2 shows schematically the motions of practitioner hands reproduced by an example of a catheter robot module according to the invention.
Fig. 3 shows schematically the correspondence between practitioner hands and the pairs of movable pads of an example of a catheter robot module according to the invention.
Fig. 4 shows schematically the different phases of the motions of two pads belonging to a pair of movable pads in an example of a catheter robot module according to the invention.
Fig. 5 shows schematically an example of a shape of a graph giving a targeted position of a pair of movable pads along a longitudinal axis x as a function of time, the flexible elongated medical element moving along this longitudinal axis x, in an example of a catheter robot module according to the invention.
Fig. 6 shows schematically an example of a correspondence between a shape of a graph giving a targeted position of a pair of movable pads along a longitudinal axis x as a function of time, the flexible elongated medical element moving along this longitudinal axis x, and a shape of a graph giving an actual position of a pair of movable pads along a longitudinal axis x as a function of time, in an example of a catheter robot module according to the invention.
Fig. 7 shows schematically an example of a shape of a graph giving a more realistic targeted position of a pair of movable pads along a longitudinal axis x as a function of time, the flexible elongated medical element moving along this longitudinal axis x, in an example of a catheter robot module according to the invention.
Fig. 8 shows schematically the evolution of clamping curve of the flexible elongated medical element between two movable pads of one of the pairs of movable pads, as a function of time, in an example of a catheter robot module according to the invention.
Fig. 9 shows schematically the moves of the two pads of a pair of movable pads, within the horizontal plane, in an example of a catheter robot module according to the invention.
Fig. 10 shows schematically the different phases of a rotation of a flexible elongated medical element between two pads of a pair of movable pads, in an example of a catheter robot module according to the invention.
Fig. 11 shows schematically graphs of evolution, as a function of time, of clamping states of the two pairs of movables pads, for a translation of the flexible elongated medical device, in an example of a catheter robot module according to the invention.
Fig. 12 shows schematically graphs of evolution, as a function of time, of clamping states of the two pairs of movables pads, for a slow rotation of the flexible elongated medical device, in an example of a catheter robot module according to the invention.
Fig. 13 shows schematically graphs of evolution, as a function of time, of clamping states of the two pairs of movables pads, for a fast translation of the flexible elongated medical device, in an example of a catheter robot module according to the invention.
Fig. 14 shows schematically graphs of evolution, as a function of time, of clamping state of one of the pairs of movables pads, for a combined translation and rotation of the flexible elongated medical device, in an example of a catheter robot module according to the invention.
Fig. 15 shows schematically graphs of evolution, as a function of time, of clamping state of the other one of the pairs of movables pads, for a combined translation and rotation of the flexible elongated medical device, in an example of a catheter robot module according to the invention.
Fig. 16 shows schematically graphs of evolution, as a function of time, of clamping state of both pairs of movables pads, for a combined translation and rotation of the flexible elongated medical device, in an example of a catheter robot module according to the invention.
Fig. 17 shows schematically graphs of evolution, as a function of time, of translation of the flexible elongated medical device, with a sudden change of user speed setpoint, in an example of a catheter robot module according to the invention.
Fig. 18 shows schematically graphs of evolution, as a function of time, of translation of the flexible elongated medical device, with a change of user speed setpoint, with a desynchronization problem with a fixed duration of U-turn, in an example of a catheter robot module according to the invention.
Fig. 19 shows schematically a graph of evolution, as a function of time, of translation of the flexible elongated medical device, with a first margin, in an example of a catheter robot module according to the invention.
Fig. 20 shows schematically a graph of evolution, as a function of time, of translation of the flexible elongated medical device, with use of the first margin, with a positive user setpoint, in an example of a catheter robot module according to the invention.
Fig. 21 shows schematically a graph of evolution, as a function of time, of translation of the flexible elongated medical device, with use of the first margin, with a negative user setpoint, in an example of a catheter robot module according to the invention.
Fig. 22 shows schematically a graph of evolution, as a function of time, of translation of the flexible elongated medical device, with use of a second margin, with a positive user setpoint, in an example of a catheter robot module according to the invention.
Fig. 23 shows schematically graphs of evolution, as a function of time, of translation of the flexible elongated medical device, with synchronized and unsynchronized moves, in an example of a catheter robot module according to the invention.
Fig. 24 shows schematically graphs of evolution, as a function of time, of translation of the flexible elongated medical device, with a first step of correction of desynchronization, in an example of a catheter robot module according to the invention.
Fig. 25 shows schematically graphs of evolution, as a function of time, of translation of the flexible elongated medical device, with a second step of correction of desynchronization, in an example of a catheter robot module according to the invention.
Fig. 26 shows schematically graphs of evolution, as a function of time, of translation of the flexible elongated medical device, with a third step of correction of desynchronization, in an example of a catheter robot module according to the invention.
Fig. 27 shows schematically graphs of evolution, as a function of time, of translation of the flexible elongated medical device, with a fourth step of correction of desynchronization, in an example of a catheter robot module according to the invention.
Fig. 28 shows schematically a graph of evolution, as a function of user speed setpoint, of a wished U-turn duration, in an example of a catheter robot module according to the invention.
Fig. 29 shows schematically graphs of evolution, as a function of time, of translation of the flexible elongated medical device, with a step of management of clamping conflict, in an example of a catheter robot module according to the invention.
Fig. 30 shows schematically graphs of evolution, as a function of time, of translation of the flexible elongated medical device, with another step of management of clamping conflict, in an example of a catheter robot module according to the invention.
Fig. 31 shows schematically graphs of evolution, as a function of time, of translation of the flexible elongated medical device, with a progressive change of user speed setpoint, in an example of a catheter robot module according to the invention.
Fig. 32 shows schematically graphs of evolution, as a function of time, of translation of the flexible elongated medical device, with a change of user speed setpoint during the U-turn, in an example of a catheter robot module according to the invention.
Fig. 33 shows schematically the temporal evolution of four finite state machines, in an example of a catheter robot module according to the invention.
Fig. 34 shows schematically the 12 states of a cycle of the four finite state machines, in an example of a catheter robot module according to the invention.
Fig. 35 shows schematically a synoptic representing a cycle of the four finite state machines, in an example of a catheter robot module according to the invention.

### DETAILED DESCRIPTION OF THE INVENTION

The present invention deals with a catheter robot module implementing a process used to coordinate the motion of the actuators in a robotic module designed to move a guidewire or any flexible elongate medical element. It is intended to be part of a robot manipulating flexible elongated medical elements (guidewires, balloon or stent catheters, guiding catheters, etc.) for vascular interventions in various domains (interventional cardiology, interventional neuroradiology, peripheral vascular interventions, etc...). Such a catheter robot module is described in more detail in WO2015189531.

Embodiments of the invention relate to a process to control the motions of a robotic platform designed for the manipulation of at least one flexible elongated medical element in the vascular field, said robotic platform containing:
- A control unit allowing a user to set the translation and/or rotation speed setpoints of the at least one flexible elongated medical element, in a continuous way,
- A robot, which can communicate with the control unit to receive continuously in real time said translation and/or rotation speed setpoints, and which contains at least one robotic module, said robotic module containing:
   ∘ At least two pairs of fingers manipulating the at least one flexible elongated medical element:
      ▪ Each finger being able to move along the x, y and z axis,
      ▪ Each pair of fingers having linked movement along each axis:
         - X axis (control of translation): identical movements,
         - Y axis (control of clamping): opposite movements in disjointed range, the distance between the maximum position of the finger with the lower range and the minimum position of the finger with the higher range being approximately equal to the diameter of the flexible elongated medical element,
         - Z axis (control of rotation): opposite movements in the same range, i.e. when one finger is at the maximum position, the other finger is at the minimum position and vice-versa,
            to move said flexible elongated medical element in translation and/or rotation according to said translation and/or rotation speed setpoints defined by the user,
   ∘ Motors,
   ∘ Mechanical interfaces between the motors and the at least two pairs of fingers,
   ∘ A sterile interface placed between the at least two pairs of fingers and the at least one flexible elongated medical element,
   ∘ Electronics and embedded software controlling the motors,
   ∘ Four Finite State Machines (FSM) to control the motion of the at least two pairs of fingers, through the control of the motors and thanks to the motion transmission of the mechanical interfaces:
      ▪ The first FSM controlling the motion of the first pair of fingers along the x and y axis (translation of the flexible elongated medical element),
      ▪ The second FSM controlling the motion of the first pair of fingers along the z and y axis (rotation of the flexible elongated medical element),
      ▪ The third FSM controlling the motion of the second pair of fingers along the x and y axis (translation of the flexible elongated medical element),
      ▪ The fourth FSM controlling the motion of the second pair of fingers along the z and y axis (rotation of the flexible elongated medical element),
   ∘ Each FSM:
      ▪ has at least the 4 following phases:
         1. Clamping phase: Clamping the flexible elongated medical element,
         2. Active phase: Moving the flexible elongated medical element in translation (first and third FSM) or rotation (second or fourth FSM) according to the user defined translation and/or rotation speed setpoints,
         3. Unclamping phase: Unclamping the flexible elongated medical element,
         4. U-turn phase: While remaining unclamped, going back to (or near) the initial position of phase 1,
      ▪ has a "margin mechanism", activated during the phase 1, using a "standard" travel range and an "extended" travel range, where a pair of finger uses normally the "standard" range, but continues on the "extended" range in case the other hand is temporarily not clamping the flexible elongated medical element, in order to ensure that at least one of the two pairs of fingers is always clamped,
      ▪ has a "U-turn duration adaptation mechanism", activated during phase 4, based on adaptation of the duration of said "U-turn phase", to ensure that the motion along the x axis (for first and third FSM) or z axis (for second and fourth FSM) of the pair of fingers is maintained in phase opposition with the same motion of the other pair, so that there is an optimized cooperation between the two pairs,
   ∘ where the potential conflict due to both the first and second FSM controlling the motion of the first pair of fingers along the y axis and of both the third and fourth FSM controlling the motion of the second pair of fingers along the y axis is solved in the following way: If one FSM asks for clamping and the other asks for unclamping, then unclamp.

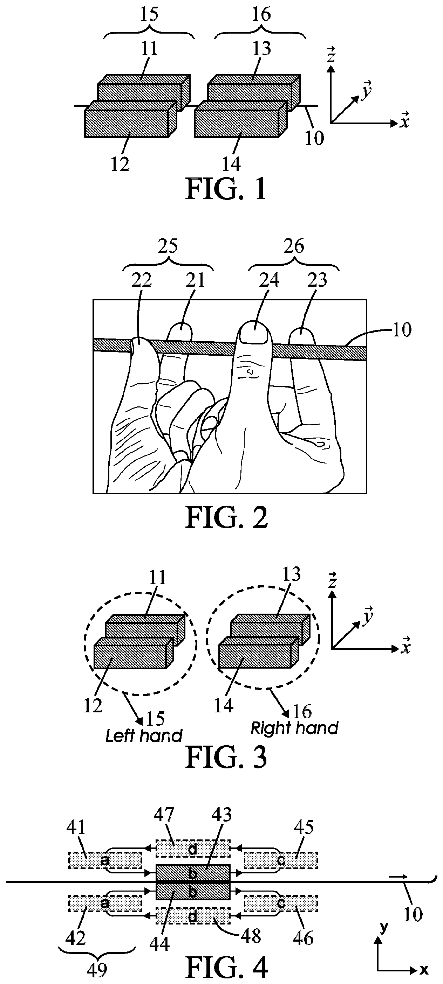

Fig. 1 shows schematically two pairs of two movable pads each, within an example of a catheter robot module according to the invention.

A robotic module is composed of 4 pads 11, 12, 13 and 14, each of them being able to move in 3 directions (x, y, z). there are a first pair 15 of pads 11 and 12, and a second pair 16 of pads 13 and 14. Pads 11 to 14 first clamp a flexible elongated medical element 10, and second translate and/or rotate this flexible elongated medical element 10.

Fig. 2 shows schematically the motions of practitioner hands reproduced by an example of a catheter robot module according to the invention.

These pads 11 to 14 are equivalent to 4 fingers 21, 22, 23 and 24, manipulating a tube 10 as shown on figure 2. The tube is translated and rotated as would do fingers 21 and 22 of left hand 25 as well as fingers 23 and 24 of right hand 26.

Fig. 3 shows schematically the correspondence between practitioner hands and the pairs of movable pads of an example of a catheter robot module according to the invention.

As in the figure 2, there are two pairs 15 and 16, respectively of pads 11 and 12, and 13 and 14. These two pairs 15 and 16 will be called them the "left hand" 15 and the "right hand" 16. More generally speaking, "hand" will refer to a pair of pads.

Fig. 4 shows schematically the different phases of the motions of two pads belonging to a pair of movable pads in an example of a catheter robot module according to the invention.

The translation motion is obtained thanks to combined motions of the pads in the x and y directions. The figure 4 illustrates this motion on one hand, what means on one pair of pads.

The following steps are repeated:
➢ Phase a: clamping of pads 41 and 42, (by an y axis motion,
➢ Phase b: translation of pads 43 and 44, by an x axis motion,
➢ Phase c: unclamping of pads 45 and 46, by an y axis motion,
➢ Phase d: go back to initial position or "U-turn", of pads 47 and 48, by an x axis motion.

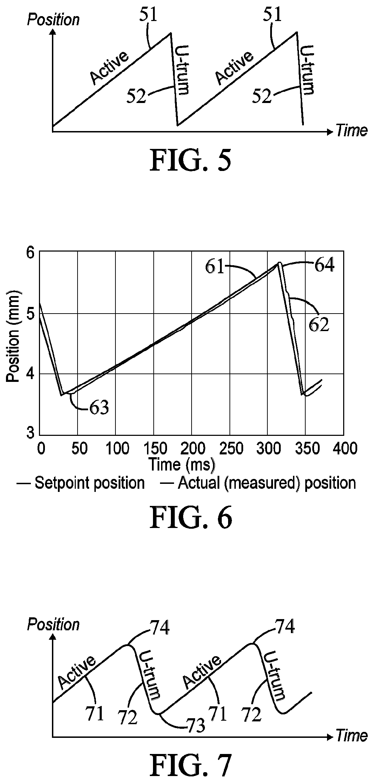

Fig. 5 shows schematically an example of a shape of a graph giving a targeted position of a pair of movable pads along a longitudinal axis x as a function of time, the flexible elongated medical element moving along this longitudinal axis x, in an example of a catheter robot module according to the invention.

During the "active" phase 51, the pads 11 and 12 for instance, and/or pads 13 and 14 as may be the case, are clamped and the speed of these pads along the x axis corresponds to the wanted flexible elongated medical element speed, as defined by the user from the control unit, using for example using joysticks. This corresponds to the phase b of the figure 4.

During the "U-turn" phase 52, the pads 11 and 12 are unclamped, and they travel along the x axis in the opposite direction to go back to their initial position and be ready for the next active phase 51. This corresponds to the phases c, d and a of the figure 4.

Here, a cycle with two phases is described, an active and a U-turn phase. This is a simplified cycle for the sake of clarity. This will be refined below, because a cycle actually breaks down into more than two phases.

In this example, we have represented a U-turn phase 52 with a linear path. In practice no physical system could follow such a path, because sudden speed changes would imply infinite accelerations.

Fig. 6 shows schematically an example of a correspondence between a shape of a graph giving a targeted position of a pair of movable pads along a longitudinal axis x as a function of time, the flexible elongated medical element moving along this longitudinal axis x, and a shape of a graph giving an actual position of a pair of movable pads along a longitudinal axis x as a function of time, in an example of a catheter robot module according to the invention.

The figure 6 shows the actual position of the pads 11 and 12, with active phase 63 and U-turn phase 62, both active phase 63 and U-turn phase 62 being linked by a rounded junction 64. This is to be compared with the theoretical triangular path 61.

Fig. 7 shows schematically an example of a shape of a graph giving a more realistic targeted position of a pair of movable pads along a longitudinal axis x as a function of time, the flexible elongated medical element moving along this longitudinal axis x, in an example of a catheter robot module according to the invention.

For sake of clarity, the embodiment of the invention will be described with a target path of the actuators which are triangular curves. Other embodiments are possible, and may be better, where the U-turn phase has a more "rounded" shape, as illustrated in figure 7. Figure 7 shows active phase 71 followed by U-turn phase 72, both being linked together by a rounded part 74. Such a curve will result in less effort requirements for the actuators as well as less stress for the mechanics.

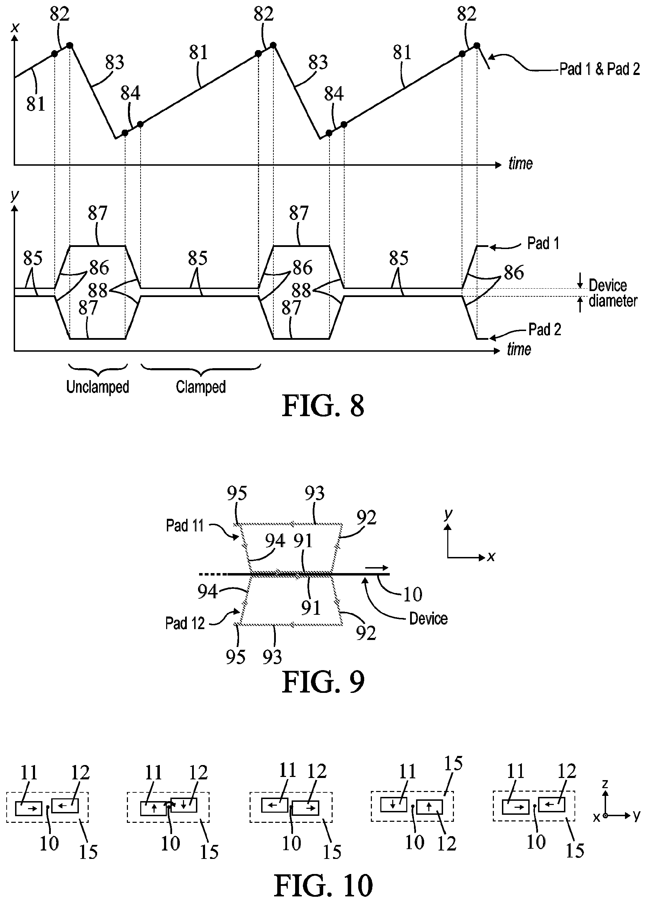

Fig. 8 shows schematically the evolution of clamping curve of the flexible elongated medical element between two movable pads of one of the pairs of movable pads, as a function of time, in an example of a catheter robot module according to the invention.

To implement the motion of the pads 11 and 12 aiming at translating a flexible elongated medical element along the x axis, the pads also need to move along the y axis to clamp and unclamp the flexible elongated medical element. These motions along two different axes have to be synchronized as illustrated in figure 8, to produce the wanted motion of the pads 11 and 12. On figure 8, the motion of the two pads, (first Pad 1 & second Pad 2) of one hand, may be seen, either pads 11 and 12 of pair 15, or pads 13 and 14 of pair 16. In a cycle, there are successively following phases: active phase 81, unclamping phase 82, U-turn phase 83, clamping phase 84, and again active phase 81 of next cycle.

The two pads 11 and 12 get closer during the clamping phase 84. They do not touch each other, though, because the flexible elongated medical element is between them: their closest distance corresponds to the flexible elongated medical element diameter, noted "device diameter" on figure 8.

The cycle of a pair 15 includes the following phases: active phase 85 translating the flexible elongated medical element with both pads 11 and 12 clamped around this flexible elongated medical element, then unclamping phase 86 with both pads 11 and 12 releasing this flexible elongated medical element, then U-turn phase 87 with pads 11 and 12 getting back towards their initial position, then clamping phase 88 with pads 11 and 12 getting closer until touching and maintaining this flexible elongated medical element, and then again active phase 85 of next cycle.

Fig. 9 shows schematically the moves of the two pads of a pair of movable pads, within the horizontal plane, in an example of a catheter robot module according to the invention.

The cycle of a pair 15 of pads 11 and 12 includes the following phases: active phase 91 translating the flexible elongated medical element with both pads 11 and 12 clamped around this flexible elongated medical element 10, then unclamping phase 92 with both pads 11 and 12 releasing this flexible elongated medical element, then U-turn phase 93 with pads 11 and 12 getting back towards their initial position, then clamping phase 94 with pads 11 and 12 getting closer until touching and maintaining this flexible elongated medical element 10, and then again active phase 91 of next cycle.

The figure 8 shows that re-clamping after the U-turn phase 87 starts with a small delay after the phase 83. This is a wanted effect in order to take into account the response time of the actuators as explained on figure 6. This results in a small "appendix" 95 on the left of the pads path, as shown on figure 9.

The maximum distance between the two pads 11 and 12 along the y axis during unclamping has been voluntarily exaggerated for a better readability. In practice, the unclamping distance should be minimized in order to reduce power consumption and increase performance.

Fig. 10 shows schematically the different phases of a rotation of a flexible elongated medical element between two pads of a pair of movable pads, in an example of a catheter robot module according to the invention.

Rotation is based on the same principle as translation, except that the motion uses the RR (right rotation) and LR (left rotation) actuators instead of RT (right translation) and LT (left translation). The flexible elongated medical element rolls between the two pads of one hand, as illustrated in figure 10.

A rotating cycle has following phases, from left to right sides of figure 10:
➢ First, pads 11 and 12 of pair 15 clamp flexible elongated medical element 10,
➢ Then, pads 11 and 12 of pair 15 translate in opposite directions to make flexible elongated medical element 10 rotate between them, like fingers making a tube or a cigarette rolling between them,
➢ Then, pads 11 and 12 of pair 15 unclamp flexible elongated medical element 10,
➢ Then, pads 11 and 12 of pair 15 go back to their initial positions with respect to flexible elongated medical element 10,
➢ Then, pads 11 and 12 of pair 15 clamp flexible elongated medical element 10, starting thereby a new rotation cycle.

Since translation and rotation motions are independent, it is possible to combine them to translate and rotate the flexible elongated medical element 10 simultaneously. The translation uses the actuators along the x axis, while simultaneously the actuators along the z axis enable the rotation.

Each hand has therefore two pads, pads 11 and 12 for pair 15 (left hand), and pads 13 and 14 for pair 16 (right hand), each of them having 3 degrees of freedom. This seems at first sight to result in 6 degrees of freedom per hand, and a total of 12 degrees of freedom.

However, some motions are linked, thus reducing the total number of degrees of freedom:
- On the x axis, both pads 11 and 12 of the same hand have the same motion,
- On the z axis, both pads 11 and 12 of the same hand have opposite motions: in order to roll the flexible elongated medical element 10 between the pads 11 and 12, one goes up while the other goes down, thereby making the flexible elongated medical element 10 rolling between the pads 11 and 12,
- On the y axis, both pads 11 and 12 of the same hand have opposite motions: in order to clamp, they have to go closer to each another, and, to unclamp, they need to move away from each other.

Since there is a coupling between the two pads on each axis, this reduces the number of degrees of freedom to 6.

The actuators will be named, by using the following abbreviations, as mentioned in table 1.

**TABLE 1**

| ***Abbreviation*** | ***Meaning*** | ***Axis*** | ***Used in flexible elongated medical element translation*** | ***Used in flexible elongated medical element rotation*** |
|---|---|---|---|---|
| RT | Right Translation | X | ✔ | |
| RR | Right Rotation | Z | | ✔ |
| RC | Right Clamping | Y | ✔ | ✔ |
| LT | Left Translation | X | ✔ | |
| LR | Left Rotation | Z | | ✔ |
| LC | Left Clamping | Y | ✔ | ✔ |

This table 1 has 6 lines, corresponding to the 6 degrees of freedom listed above. Each line therefore represents the motion of the two pads 11 and 12 (or 13 and 14) of one hand (corresponding to pair 15 or to pair 16) along one axis.

Since there is a fixed link between the motion of the two pads 11 and 12 of the same hand, there is no more need to show the motion of both pads of both hands, as on figure 8, but showing motion of one pad of each hand will be considered as sufficient.

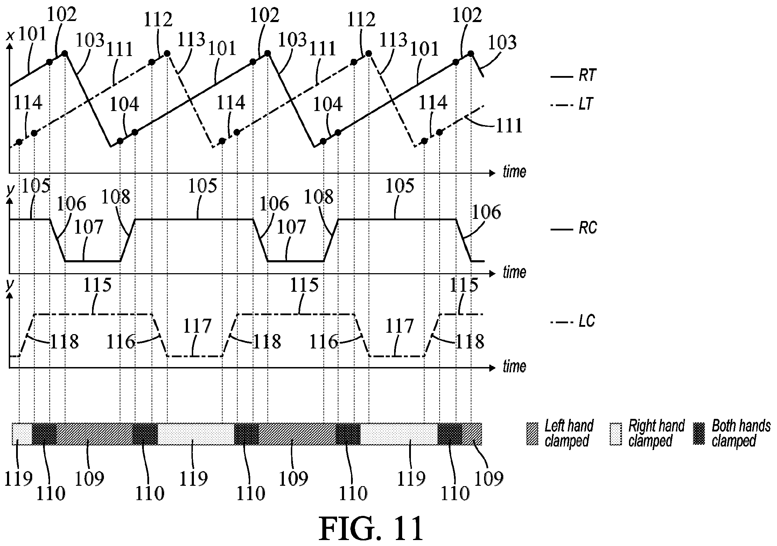

Fig. 11 shows schematically graphs of evolution, as a function of time, of clamping states of the two pairs of movables pads, for a translation of the flexible elongated medical device, in an example of a catheter robot module according to the invention.

In a cycle for pair 16 of pads 13 and 14, there are successively following phases: active phase 101, unclamping phase 102, U-turn phase 103, clamping phase 104, and again active phase 101 of next cycle.

In a cycle for pair 15 of pads 11 and 12, there are successively following phases: active phase 111, unclamping phase 112, U-turn phase 113, clamping phase 114, and again active phase 111 of next cycle.

With respect to translation motion of flexible elongated element 10, this cycle of a pair 16 of pads 13 and 14, may be seen as including the following phases: active phase 105 translating the flexible elongated medical element 10 with both pads 13 and 14 clamped around this flexible elongated medical element 10, then unclamping phase 106 with both pads 13 and 14 releasing this flexible elongated medical element 10, then U-turn phase 107 with pads 11 and 12 getting back towards their initial position, then clamping phase 108 with pads 13 and 14 getting closer until touching and maintaining this flexible elongated medical element 10, and then again active phase 105 of next cycle.

With respect to translation motion of flexible elongated element 10, this cycle of a pair 15 of pads 11 and 12, may be seen as including the following phases: active phase 115 translating the flexible elongated medical element 10 with both pads 11 and 12 clamped around this flexible elongated medical element 10, then unclamping phase 116 with both pads 11 and 12 releasing this flexible elongated medical element 10, then U-turn phase 117 with pads 11 and 12 getting back towards their initial position, then clamping phase 118 with pads 11 and 12 getting closer until touching and maintaining this flexible elongated medical element 10, and then again active phase 115 of next cycle.

Considering the figure 5, it can be seen that a hand 15 can move the flexible elongated medical element 10 only during the active phase 111, i.e. when the two pads 11 and 12 are clamped. In order to obtain a continuous motion of the flexible elongated medical element 10, both pairs 15 and 16 of pads need to cooperate. When one pair 15 is in U-turn phase 113, the other pair 16 should be in active phase 101 thus ensuring that the flexible elongated medical element 10 is being clamped and moved by at least one pair of pads at any time.

The figure 11 illustrates such a cooperation for translation. It shows the translation of both hands 15 and 16 (RT and LT) combined with clamping / unclamping of both hands 15 and 16. In order to get the best possible cooperation, the curves of one hand should be close to exact phase opposition.

As shown on the colored bar at the bottom of figure 11, there are periods where one hand 15 or 16 is clamped, respectively periods 109 or 119, and periods 110 where both hands 15 and 16 are clamped. These latter periods 110, which are also called "overlapping periods" 110, are very useful to ensure that the motion of flexible elongated medical element is quite or even perfectly fluid.

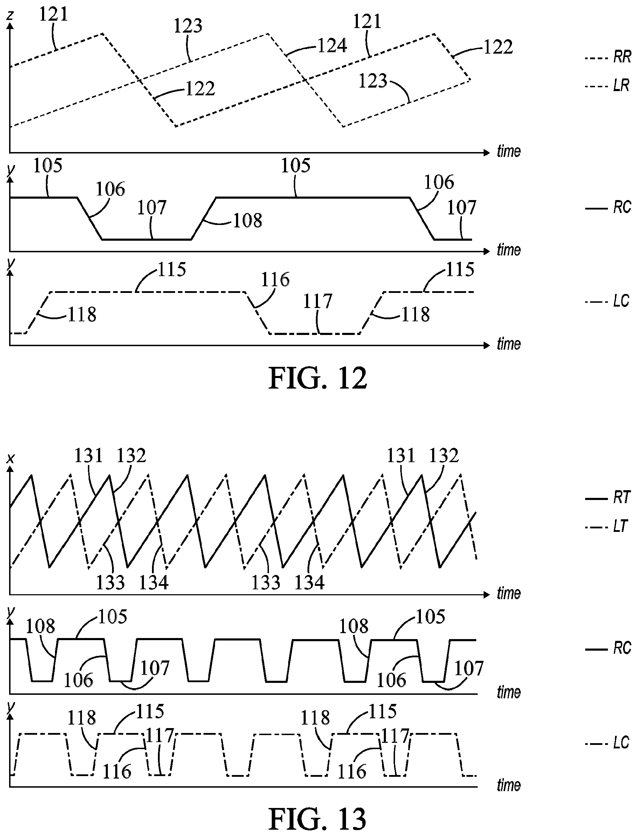

Fig. 12 shows schematically graphs of evolution, as a function of time, of clamping states of the two pairs of movables pads, for a slow rotation of the flexible elongated medical device, in an example of a catheter robot module according to the invention.

For pair 16 of pads 13 and 14, there is a succession of cycles each including succession of an active phase 121 followed by a U-turn phase 122.

For pair 15 of pads 11 and 12, there is a succession of cycles each including succession of an active phase 123 followed by a U-turn phase 124.

With respect to rotation motion of flexible elongated element 10, this cycle of a pair 16 of pads 13 and 14, may be seen as including the following phases: active phase 105 rotating the flexible elongated medical element 10 with both pads 13 and 14 clamped around this flexible elongated medical element 10, then unclamping phase 106 with both pads 13 and 14 releasing this flexible elongated medical element 10, then U-turn phase 107 with pads 13 and 14 getting back towards their initial position, then clamping phase 108 with pads 13 and 14 getting closer until touching and maintaining this flexible elongated medical element 10, and then again active phase 105 of next cycle.

With respect to rotation motion of flexible elongated element 10, this cycle of a pair 15 of pads 11 and 12, may be seen as including the following phases: active phase 115 rotating the flexible elongated medical element 10 with both pads 11 and 12 clamped around this flexible elongated medical element 10, then unclamping phase 116 with both pads 11 and 12 releasing this flexible elongated medical element 10, then U-turn phase 117 with pads 11 and 12 getting back towards their initial position, then clamping phase 118 with pads 11 and 12 getting closer until touching and maintaining this flexible elongated medical element 10, and then again active phase 115 of next cycle.

As seen previously, the same pair of pads, either pair 15 of pads 11 and 12 or pair 16 of pads 13 and 14, handles simultaneously the translation and rotation of the flexible elongated medical element, thanks to motions along the x and z axis.

In addition, it can be seen that the translation and rotation speeds are user defined and can be set in a fully independent manner from each other.

When considering both pads of both hands 15 and 16 during a simultaneous and continuous translation and rotation of a flexible elongated medical element 10, the consequence of the motion principles of the pads 11 and 12, or 13 and 14, along the various axes imply the following rules:
➢ unclamping has to be done when the pads 11 and 12, or 13 and 14, reach their maximum position along the x (for translation) or z (for rotation) axis,
➢ unclamping of both hands 15 and 16 has to be avoided since this would stop the flexible elongated medical element 10 motion. Moreover, unclamping of both hands 15 and 16 could lead to unwanted and thus unsafe movements of the flexible elongated medical element 10.

The problem lies in the potential contraction between these points. This can be seen when considering both a slow rotation as on figure 12, and a fast translation as on figure 13, with cycles of successive active phase 131 and U-turn phase 132, for pair 16 of pads 13 and 14, and cycles of successive active phase 133 and U-turn phase 134, for pair 15 of pads 11 and 12.

Fig. 13 shows schematically graphs of evolution, as a function of time, of clamping states of the two pairs of movables pads, for a fast translation of the flexible elongated medical device, in an example of a catheter robot module according to the invention.

With respect to translation motion of flexible elongated element 10, this cycle of a pair 16 of pads 13 and 14, may be seen as including the following phases: active phase 105 translating the flexible elongated medical element 10 with both pads 13 and 14 clamped around this flexible elongated medical element 10, then unclamping phase 106 with both pads 13 and 14 releasing this flexible elongated medical element 10, then U-turn phase 107 with pads 13 and 14 getting back towards their initial position, then clamping phase 108 with pads 13 and 14 getting closer until touching and maintaining this flexible elongated medical element 10, and then again active phase 105 of next cycle.

With respect to translation motion of flexible elongated element 10, this cycle of a pair 15 of pads 11 and 12, may be seen as including the following phases: active phase 115 translating the flexible elongated medical element 10 with both pads 11 and 12 clamped around this flexible elongated medical element 10, then unclamping phase 116 with both pads 11 and 12 releasing this flexible elongated medical element 10, then U-turn phase 117 with pads 11 and 12 getting back towards their initial position, then clamping phase 118 with pads 11 and 12 getting closer until touching and maintaining this flexible elongated medical element 10, and then again active phase 115 of next cycle.

Now, what happens if these two movements are to be combined? The movements along the x and z axis are independent and do not interfere with each other. However, the movements along the y axis (clamping) have a different status. Indeed, the clamping movement is linked to both translation and rotation, because, when unclamping a hand 15, both the translation and the rotation of this hand 15 are interrupted. Therefore, when translation (for example) has reached its maximum position and requests an unclamping motion, this has a side effect on rotation, which may not need unclamping at this time.

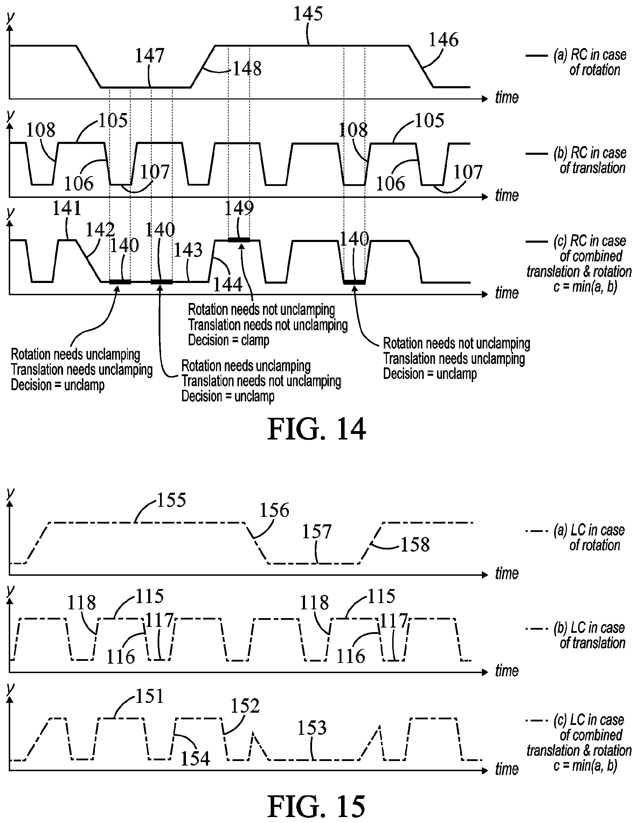

Fig. 14 shows schematically graphs of evolution, as a function of time, of clamping state of one of the pairs of movables pads, for a combined translation and rotation of the flexible elongated medical device, in an example of a catheter robot module according to the invention.

With respect to translation motion of flexible elongated element 10, this cycle of a pair 16 of pads 13 and 14, may be seen as including the following phases: active phase 105 translating the flexible elongated medical element 10 with both pads 13 and 14 clamped around this flexible elongated medical element 10, then unclamping phase 106 with both pads 13 and 14 releasing this flexible elongated medical element 10, then U-turn phase 107 with pads 13 and 14 getting back towards their initial position, then clamping phase 108 with pads 13 and 14 getting closer until touching and maintaining this flexible elongated medical element 10, and then again active phase 105 of next cycle.

With respect to rotation motion of flexible elongated element 10, this cycle of a pair 16 of pads 13 and 14, may be seen as including the following phases: active phase 145 rotating the flexible elongated medical element 10 with both pads 13 and 14 clamped around this flexible elongated medical element 10, then unclamping phase 146 with both pads 13 and 14 releasing this flexible elongated medical element 10, then U-turn phase 147 with pads 13 and 14 getting back towards their initial position, then clamping phase 148 with pads 13 and 14 getting closer until touching and maintaining this flexible elongated medical element 10, and then again active phase 145 of next cycle.

However, in a first approach, in order to allow both the translation and the rotation to do their respective U-turn phases 107 and 147, unclamping will be performed if either the translation or the rotation needs it.

To do so, in figure 14, one clamping curve (RC) in case of rotation only is combined with the same curve in case of translation only. For a combined rotation and translation, these two curves will be combined. Since, in this case, the unclamped position is the lowest, the combination will consist in calculating the minimum value of these two curves.

The result of this combination is the lowest curve of figure 14, with clamped position 141 and unclamped position 143, with clamping 144 and unclamping 142.

If both rotation and translation ask for unclamp as in zone 140, then unclamp prevails. If both rotation and translation ask for clamp as in zone 149, then clamp prevails.

Fig. 15 shows schematically graphs of evolution, as a function of time, of clamping state of the other one of the pairs of movables pads, for a combined translation and rotation of the flexible elongated medical device, in an example of a catheter robot module according to the invention.

With respect to translation motion of flexible elongated element 10, this cycle of a pair 15 of pads 11 and 12, may be seen as including the following phases: active phase 115 translating the flexible elongated medical element 10 with both pads 11 and 12 clamped around this flexible elongated medical element 10, then unclamping phase 116 with both pads 11 and 12 releasing this flexible elongated medical element 10, then U-turn phase 117 with pads 13 and 14 getting back towards their initial position, then clamping phase 118 with pads 11 and 12 getting closer until touching and maintaining this flexible elongated medical element 10, and then again active phase 105 of next cycle.

With respect to rotation motion of flexible elongated element 10, this cycle of a pair 15 of pads 11 and 12, may be seen as including the following phases: active phase 155 rotating the flexible elongated medical element 10 with both pads 11 and 12 clamped around this flexible elongated medical element 10, then unclamping phase 156 with both pads 11 and 12 releasing this flexible elongated medical element 10, then U-turn phase 157 with pads 11 and 12 getting back towards their initial position, then clamping phase 158 with pads 11 and 12 getting closer until touching and maintaining this flexible elongated medical element 10, and then again active phase 155 of next cycle.

The result of this combination is the lowest curve of figure 14, with clamped position 151 and unclamped position 153, with clamping 154 and unclamping 152.

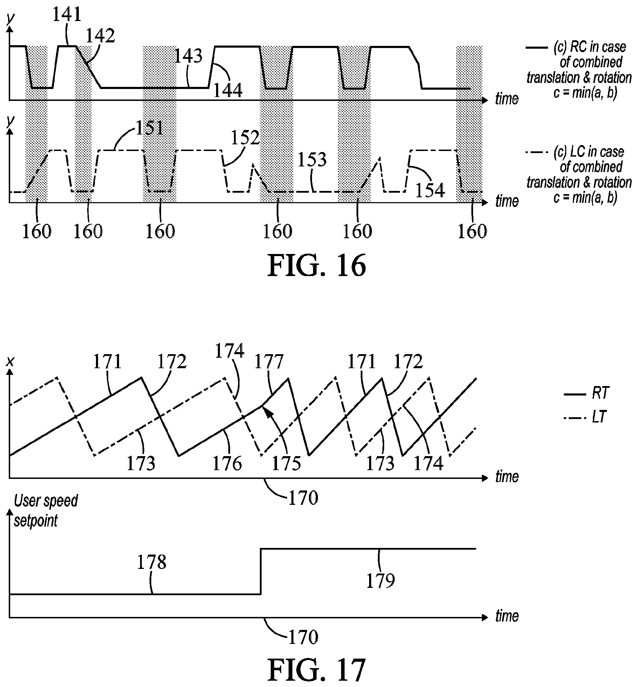

If the RC curve of clamping in case of combined translation and rotation from figure 14, and the LC curve of clamping in case combined translation and rotation from figure 15 are now extracted, this leads to get at figure 16.

Fig. 16 shows schematically graphs of evolution, as a function of time, of the wanted clamping state of both pairs of movables pads, for a combined translation and rotation of the flexible elongated medical device, in an example of a catheter robot module according to the invention.

In this figure 16, the periods of time 160 when both the left hand 15 and the right hand 16 are unclamped (RC and LC low) have been highlighted. This is a problem since this corresponds to both pairs 15 and 16 simultaneously unclamped, what is to be avoided, since these periods 160 lead to loss of control over flexible elongated medical element 10 and to idle periods 160 where no motion can be imparted to flexible elongated medical element 10 , whether translation motion or rotation motion.

During combined translation and rotation, the path of the pads along the x and z axis cannot behave independently without inconvenience, only considering the user speed setpoint. But, on the contrary, the translation (along the x axis) has to take into account what is happening on rotation (along the z axis), and vice-versa.

Fig. 17 shows schematically graphs of evolution, as a function of time, of translation of the flexible elongated medical device, with a sudden change of user speed setpoint, in an example of a catheter robot module according to the invention.

The translation of the pair of pads of one hand should be in phase opposition with the pads of the other hand (RT and LT). The same applies for rotation (RR and LR).

This should remain true even in the case of user speed setpoint change.

An apparently simple solution to this problem could be the following: the speed during the U-turn phase should change proportionally to the speed of the active phase, this latter speed being determined by the user). As can be seen on figure 17, the coordinated motion of the pads of the two hands 16 and 15 for translation (RT and LT).

For pair 16 of pads 13 and 14, active phase 171 is followed by U-turn phase 172.

For pair 15 of pads 11 and 12, active phase 173 is followed by U-turn phase 174.

In the middle of the curve, at time 170, the user changes the speed setpoint from a slower value 178 to a faster value 179. There is a corresponding change in slope 175 from smoother slope 176 to steeper slope 177, and the period of the cycles becomes shorter.

Using a U-turn speed which is proportional to the active phase speed results in a synchronization between hands 16 and 15 that is maintained after the user speed setpoint change.

However, this method would have two problems.

The first problem would be related to the lower speeds. As already seen earlier, when the speed in the U-turn phase 172 or 174 is proportional to the speed in the active phase 171 or 173, with for example a slow rotation speed which is combined with a fast translation speed, a clamping conflict is created, and thus, in short, it doesn't work efficiently.

The second problem would be related to higher speeds. In this case, the actuators will be limited, because very high actuator speeds and acceleration imply bigger actuators with more heat dissipation. In order to keep dimensions and cooling measures within reasonable limits, it is needed to put a maximum speed limit for the U-turn phase 172 or 174.

Those two problems can be merged into one statement: it is hard and it may even become not possible to set the U-turn speed as a value being proportional to the active speed, at least not without great inconvenience.

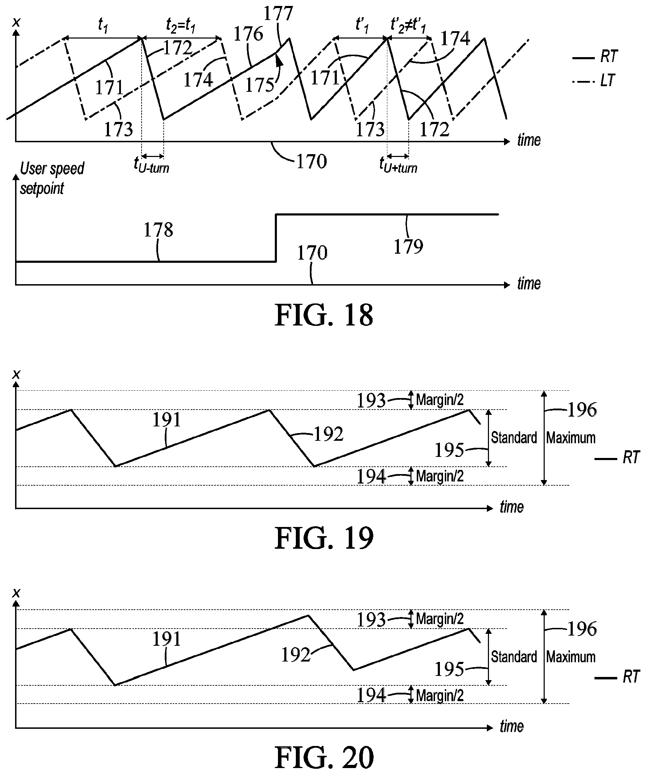

Fig. 18 shows schematically graphs of evolution, as a function of time, of translation of the flexible elongated medical device, with a change of user speed setpoint, with a desynchronization problem with a fixed duration of U-turn, in an example of a catheter robot module according to the invention.

A tentative solution could be to use a constant U-turn speed. This could be set it as high as possible, in a tentative to minimize the clamping conflict. The curves of the figure 17 would then change into those of figure 18.

However, this results in a synchronization loss between the hands: at the beginning, the hands 16 and 15 are synchronized (*t₁ = t₂*), but after the user speed setpoint change, synchronization between both hands 16 and 15 is lost (*t'₁ ≠ t'₂*).

Therefore, there is a need for a mechanism to control and maintain synchronization between the two hands 16 and 15.

Fig. 19 shows schematically a graph of evolution, as a function of time, of translation of the flexible elongated medical device, with a first margin, in an example of a catheter robot module according to the invention.

In the cycle, active phase 191 is followed by U-turn phase 192. Extension of the travel range along x axis, direction of translation of flexible elongated medical element 10, is usually a standard range 195, but in some cases it can travel within a maximum range 196 which adds a margin, split into two half margins, upper half margin 193 and lower half margin 194, added at each end of the standard range 195.

There is a similar margin for extension of the travel range along z axis, axis used to impart rotation to the flexible elongated medical element 10.

In order to avoid any clamping conflict, the travel range along the x and z axis is split between a standard range 195 and a maximum range 196, the difference between the two being the margin. Consider the curve for one movement, for example RT. In this example, the standard range 195 is used:
Fig. 20 shows schematically a graph of evolution, as a function of time, of translation of the flexible elongated medical device, with use of the first margin, with a positive user setpoint, in an example of a catheter robot module according to the invention.

This gives a margin for the translation. When reaching the maximum value of the standard range 195, if the other hand is clamped, then the U-turn can be started normally. But if the other hand is unclamped, then the upper half margin 193 between the maximum range 196 and the standard range 195 can be used in order to give time to the other hand to finish its U-turn phase, as can be seen on figure 20.

In this figure 20, can be seen such a "special" cycle where the pads travel further along the x axis in order to give time to the other hand doing its U-turn. Then the U-turn is done, with two key characteristics:
➢ The travel range of U-turn phase is always the same, which means that is has the same amplitude as the amplitude of the standard range 195,
➢ The duration of U-turn phase is defined to keep synchronization between hands 16 and 15, as is explained below.

As a result, the hands synchronization is maintained here, because the RT signal returns to its original path once the "special" cycle is finished.

Therefore, this enables to solve the clamping conflict without interfering with the hand synchronization control, which is now explained below.

The margin has to be dimensioned so as to avoid a situation where the clamping conflict would still not be solved when the pad reaches the maximum position of the maximum range 196. This will depend on system parameters: maximum translation speed, maximum rotation speed, minimum and maximum U-turn speed (for translation and rotation), etc...

In figure 8, it can be seen that unclamping has to start before the pad reaches its maximum position. This small delay is needed by the response time of the clamping/unclamping actuators. Therefore, the decision to start a U-turn has also to be anticipated by the same delay.

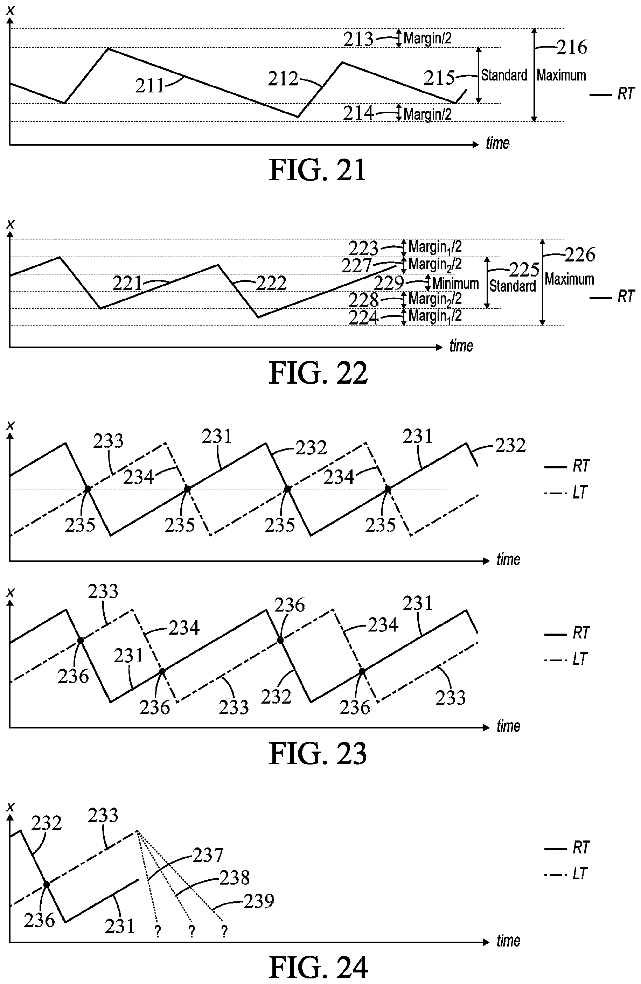

Fig. 21 shows schematically a graph of evolution, as a function of time, of translation of the flexible elongated medical device, with use of the first margin, with a negative user setpoint, in an example of a catheter robot module according to the invention.

The figure 20 illustrates the case of a positive user speed setpoint. The margin is split between a lower half margin 194 and an upper half margin 193. In this case, only the upper half margin 193 is used. In case of a negative used speed setpoint, the lower half margin 194 is used, as can be seen on figure 21.

Fig. 22 shows schematically a graph of evolution, as a function of time, of translation of the flexible elongated medical device, with use of a second margin, with a positive user setpoint, in an example of a catheter robot module according to the invention.

To summarize, the system has a tolerance on U-turn phase starting time, allowing to have a delay on it, compared to the ideal starting time.

In another embodiment, we can also allow the system to start the U-turn phase in advance. When considering the movements of one hand, the movements along the x (translation) and z (rotation) axis are usually not synchronized, as can be seen for example on figures 12 and 13, therefore leading to potentially longer unclamped periods, as can be seen on figure 15, which, in turn, could cause a clamping conflict, as shown on figure 16. Being able to anticipate a U-turn would then help limiting the unclamped periods. If, for example, the z movement starts its U-turn, and therefore triggers an unclamping of the hand, and, at the same time, the x movement is clamped but close "enough" to the end of its range, i.e. the point where it would also start a U-turn, it could then be wiser for the x movement to take advantage of the fact that the hand is unclamped to start its own U-turn, thus reducing the overall time during which the hand is unclamped.

This is shown on figure 22, where the notion of minimum range 229 has been added. Therefore, between the minimum range 229 and the standard range 225, the rule could become to start a U-turn if the hand is already unclamped (due to the other movement, i.e. x for z, or z for x). The behavior between the standard range 225 and the maximum range 226 remains the same as described previously. In the figure 22, the minimum range 229 is equal to the standard range 225 minus a first margin (being the sum of upper half margin 223 and lower half margin 224), and the maximum range 226 is equal to the standard range 225 plus a second margin (being the sum of upper half margin 227and lower half margin 228). First margin and second margin are equal on figure 22, but they could also be different from each other.

Fig. 23 shows schematically graphs of evolution, as a function of time, of translation of the flexible elongated medical device, with synchronized and unsynchronized moves, in an example of a catheter robot module according to the invention.

Pair 16 of pads 13 and 14 follows a cycle of active phase 231 and U-turn phase 232.

Pair 15 of pads 11 and 12 follows a cycle of active phase 233 and U-turn phase 234.

Either pairs 16 and 15 are synchronized with each other and their paths crossings 235 are periodical and happen at half travel extension, or pairs 16 and 15 are not synchronized with each other and their paths crossings 236 are not periodical and do not happen at half travel extension.

Adapting the synchronization between hands 16 and 15 could be compared with a phase lock loop control (PLL) mechanism, aiming at controlling the phase of a slave signal from the phase of master signal. In short, the algorithm for the slave signal could more or less amount to the following: "if you are late, accelerate, if you have advance, slow down".

The speed of the pads along the x (translation) and z (rotation) axis during the active phases 231 or 233 cannot be chosen because there are imposed by the user speed settings. However, the U-turn speed can be chosen. The travel range of the U-turn phase has to be kept constant, as seen above, so the U-turn duration will be variated to control synchronization between pairs 16 and 15.

Therefore, when needed, "accelerate" or "slow down" will be based on adjustments of the U-turn duration.

Each time a hand 16 or 15 starts a U-turn phase, it acts as a slave that has to synchronize itself with the other hand 15 or 16, which is then the master. So, the master-slave scheme is different here, because each hand 16 or 15 acts alternatively as a master and a slave.

Calculation of this U-turn duration will now be described below.

First of all, when observing synchronized (paths crossings 235) vs unsynchronized signal (paths crossings 236), it can be seen that in the first synchronized case the curves cross always at the same value (paths crossings 235), whereas unsynchronized curves cross alternatively at a higher and lower level (paths crossings 236).

Fig. 24 shows schematically graphs of evolution, as a function of time, of translation of the flexible elongated medical device, with a first step of correction of desynchronization, in an example of a catheter robot module according to the invention.

When starting from an unsynchronized situation (paths crossings 236), as illustrated on figure 24, it can be seen that LT curve has reached its maximum value, and that there is no clamping conflict so that the U-turn phase can now start. *t_{U-turn}* has to be now calculated. Different values would yield different paths, as represented in different dotted lines 237 or 238 or 239.

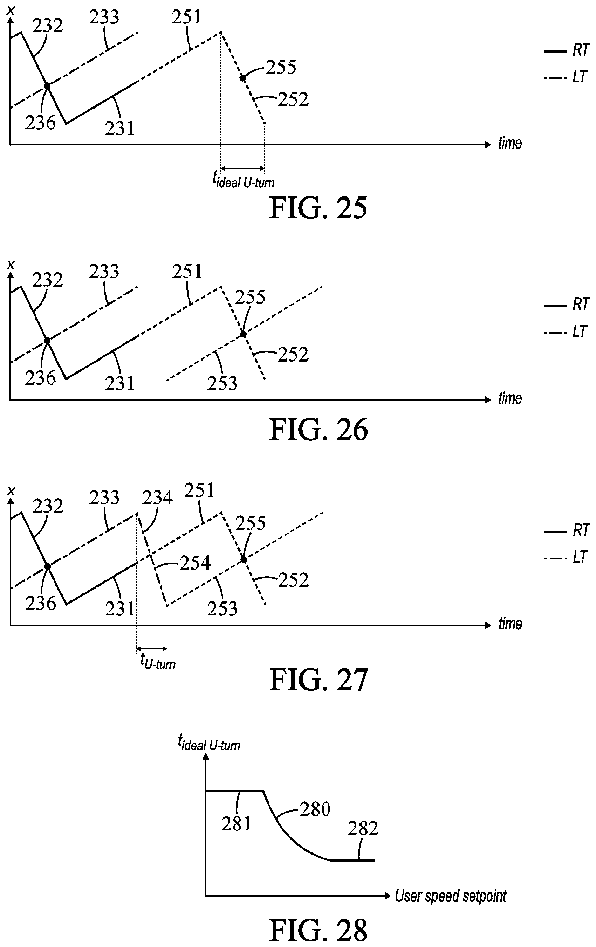

Fig. 25 shows schematically graphs of evolution, as a function of time, of translation of the flexible elongated medical device, with a second step of correction of desynchronization, in an example of a catheter robot module according to the invention.

First of all, an extrapolation can be done as to what the RT curve should look like during the next cycle: dotted line 251 for the rest of active phase and then dotted line 252 for the U-turn phase with a supposed path crossing 255 at middle travel extension with the LT curve.

Fig. 26 shows schematically graphs of evolution, as a function of time, of translation of the flexible elongated medical device, with a third step of correction of desynchronization, in an example of a catheter robot module according to the invention.

For that, the *t_{U-turn}* value of the next cycle is also extrapolated, which is called *t_{ideal U-turn}.* Calculation of *t_{ideal U-turn}* will be explained in more detail below.

The next active phase of the LT curve can now be drawn by dotted line 253, because the slope is known, since it only depends on the user speed setpoint, and it has to cross the supposed path crossing 255 because this would allow for resynchronization between both pairs 16 and 15, via the resynchronization of LT curve with the RT curve.

Fig. 27 shows schematically graphs of evolution, as a function of time, of translation of the flexible elongated medical device, with a fourth step of correction of desynchronization, in an example of a catheter robot module according to the invention.

Linking existing segments 233 and 253 will give the path of the U-turn phase 254 and therefrom the value of *t_{U-turn}* can be got directly (projection on time axis to the travel extension of U-turn phase 254).

Fig. 28 shows schematically a graph of evolution, as a function of user speed setpoint, of a wished U-turn duration, in an example of a catheter robot module according to the invention.

To calculate the next *t_{ideal U-turn},* an arbitrary function depending only on the user translation and rotation speed setpoints is used. In other words, it is the *t_{U-turn}* value that would be got in case of no need to re-synchronize the two hands 16 and 15. This function can be chosen in an arbitrary manner. An example of embodiment could be the one as represented on figure 28.

In this example, the faster the user speed setpoint, the shorter *t_{ideal U-turn}.* Indeed, for a high speed, it is needed to do the U-turn fast.

In the curved part 280, *t_{ideal U-turn}* is inversely proportional to the user speed setpoint. This means that the U-turn speed will be proportional to the user speed setpoint.

The two horizontal parts 281 and 282 have specific respective functions. In the lower user speed setpoint values, lower horizontal part 282 prevents the algorithm from using too high *t_{ideal U-turn}* values, which would lead to long periods with one hand unclamped and thus difficulties to handle clamping conflicts. On the other side of the curve, thanks to the upper horizontal part 281, there is a minimum *t_{ideal U-turn}* value that is needed because the actuators are limited in speed and could not follow, at least not easily, the requested path if *t_{ideal U-turn}* were too small.

Other curves are possible but they have to be decaying as a function of the user speed setpoint.

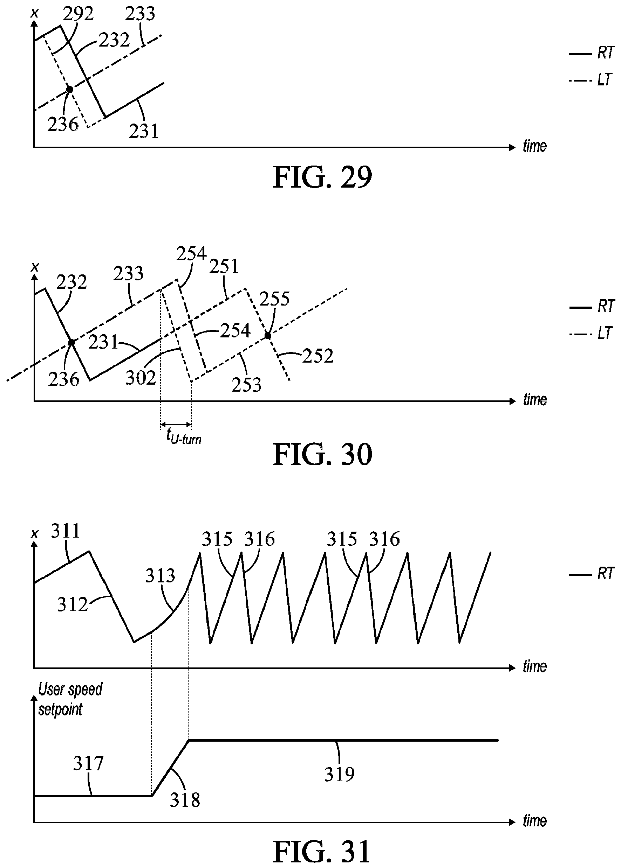

Fig. 29 shows schematically graphs of evolution, as a function of time, of translation of the flexible elongated medical device, with a step of management of clamping conflict, in an example of a catheter robot module according to the invention.

In case the last RT cycle had a clamping conflict and thus used more amplitude than the standard range, the position of the path crossing 236 using an extrapolated RT curve (dotted line 292) has to be calculated, i.e. what the RT curve would have been in case of no conflict.

Fig. 30 shows schematically graphs of evolution, as a function of time, of translation of the flexible elongated medical device, with another step of management of clamping conflict, in an example of a catheter robot module according to the invention.

A similar extrapolation is also needed in case the current LT cycle has a conflict. So, in case the last LT cycle had a clamping conflict and thus used more amplitude than the standard range, the position of the path crossing using an extrapolated LT curve (dotted line 302) has to be calculated, i.e. what the LT curve would have been in case of no conflict.

Now that *t_{U-turn}* has been calculated, a minimum value and a maximum value have to be applied. Indeed, the calculation steps detailed above do not prevent from excessively small or high values, which may have same drawbacks as the ones explained above to justify the horizontal parts 281 and 282 on figure 28. In a preferred embodiment, the minimum value is chosen smaller than the minimum of the function represented on figure 28, and the maximum greater than its function maximum.

If those minimum and maximum limits have been hit, it can be seen that the resulting *t_{U-turn}* value will then not provide synchronization immediately back, because in this case several cycles will be needed to catch up.

The term "user speed setpoint" has been used without specifying whether it is translation or rotation. Actually, it combines both. In case of a combined translation and rotation, the *t_{U-turn}* value is calculated using the function of figure 28 (or any other embodiment of such as function), for both translation and rotation, yielding two *t_{U-turn}* values: *t*_{*U-turn*_*T*} and *t_{U-turn} R.*

The minimum of the two: *t_{U-turn}* = *min*(*t_{U-turn_T}, t_{U-turn_R)}* is then used.

This is because the same unclamping movement is used for translation and rotation. A slow active phase can be combined with a fast U-turn phase, but the opposite is false, because it would cause relatively long unclamping period and create more clamping conflict possibilities. In an extreme situation where the U-turn phase would be longer than the active phase, this could run the risk to lead to having a permanent clamping conflict.

The whole synchronization process could be summarized roughly as follows. The left hand 15 synchronizes on right hand 16, then the right hand 16 synchronizes on left hand 15, etc... This process is repeated indefinitely and could lead to some instabilities, the two hands 16 and 15 "fighting" against each other. As a result, synchronization would run the risk of never being fully obtained and could somewhat oscillate between "advance" and "delay". In order to overcome this problem, in a preferred embodiment, only a fraction of the correction could be applied. *t_{calculated U-turn}* the *t_{U-turn}* value are calculated according to the method detailed above. Then, the correction factor is: *t_{correction}* = *t_{calculated U-turn} - t_{ideal U-turn}.* Applying part of the correction then means using this formula: the *t_{U-turn}* = *t_{ideal U-turn} +* α. *t_{correction},* where a is an arbitrary factor: 0 < α ≤ 1. Any value of a is possible. Lower values give better stability, higher values require less cycles to get synchronization back. In a preferred embodiment α = ½ Is chosen. This calculation is to be applied before the last step where *t_{U-turn}* is limited between minimum and maximum values.

For sake of clarity, it has been assumed on many figures, that the user speed setpoints are constant. In practice, this may not be the case, as the user could frequently change translation and rotation speed of the manipulated flexible elongated medical elements. By the way, Human-Man Interfaces allow the user to change those speed in a continuous way.

Fig. 31 shows schematically graphs of evolution, as a function of time, of translation of the flexible elongated medical device, with a progressive change of user speed setpoint, in an example of a catheter robot module according to the invention.

For pair 16 of pads 13 and 14, active phase 311 is followed by U-turn phase 312. In the middle of the curve, during a period 318, the user progressively changes the speed setpoint from a slower value 317 to a faster value 319. There is a corresponding progressive change in slope of active phase 313, past cycles presenting an active phase 311 and a U-turn phase 312 with a smoother slope, whereas future cycles will present an active phase 315 and a U-turn phase 316 with steeper slope, the period of the cycles becoming shorter.

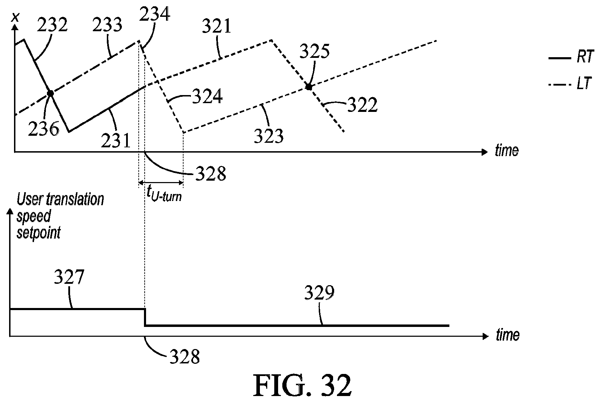

Fig. 32 shows schematically graphs of evolution, as a function of time, of translation of the flexible elongated medical device, with a change of user speed setpoint during the U-turn, in an example of a catheter robot module according to the invention.

For pair 16 of pads 13 and 14, active phase 231 is followed by U-turn phase 232.

For pair 15 of pads 11 and 12, active phase 233 is followed by U-turn phase 234.

In the middle of the curve, at a time 328, the user suddenly changes the speed setpoint from a slower value 327 to a faster value 329.

There is a corresponding sudden change in slope of U-turn phase 234, past cycles presenting:
➢ for pair 16, an active phase 231 and a U-turn phase 232 with a steeper slope, whereas future cycles will present an active phase 321 and a U-turn phase 322 with smoother slope, the period of the cycles becoming longer,
➢ for pair 15, an active phase 233 and a U-turn phase 234 with a steeper slope, whereas future cycles will present an active phase 323 and a U-turn phase 324 with smoother slope, the period of the cycles becoming longer,
➢ both pairs 16 and 15 becoming resynchronized, as can be seen through synchronized new path crossing 325, whereas before they were desynchronized as could be seen through desynchronized old path crossing 236.

On figure 31, a user speed setpoint variation is applied to the curves, if this variation takes place during the active phase 311, then the slope of the curve has just to be modified accordingly.

If this change occurs during a U-turn phase 312, the same calculation steps (as already shown with respect to figures 25, 26 and 27) can be used, except that the extrapolated curves will have to take into account the new setpoints. This is illustrated in figure 32, in case of a reduction of the translation speed setpoint.

The two mechanisms used to solve the double problem of phase opposition control and of synchronization conflict have been explained above.

Those two mechanisms are applied to each pair of pads, either pair 16 of pads 13 and 14, or pair 15 of pads 11 and 12, and to each type of motion, i.e. generating the translation and the rotation of the flexible elongated medical element 10.

This means that those two mechanisms are applied simultaneously to 4 motions indeed:
➢ The motion of the first pair 15 of pads 11 and 12 for the translation of the flexible elongated medical element 10,
➢ The motion of the first pair 15 of pads 11 and 12 for the rotation of the flexible elongated medical element 10,
➢ The motion of the second pair 16 of pads 13 and 14 for the translation of the flexible elongated medical element 10,
➢ The motion of the second pair 16 of pads 13 and 14 for the rotation of the flexible elongated medical element 10.

This is obtained thanks to 4 Finite States Machines (FSM). Each FSM has to:
- Ensure that, when clamped (i.e. during the active phase), the pads have a linear motion and impart the correct speed to the flexible elongated medical element 10 (i.e. the user setpoint speed),
- Choose when to quit the active phase and start the U-Turn phase, using the "margin mechanism" as explained in figures 19 to 22.
- Determine the initial U-turn duration, and thus the U-turn speed, using the "U-turn duration adaptation mechanism" as explained above.
- Adapt the U-turn speed in real-time during the U-turn phase, by refreshing the U-turn speed calculation, using the same "U-turn duration adaptation mechanism" as explained above.

The table 2 below lists the characteristics of these 4 FSM:

**TABLE 2**

| FSM name | ***FSM_{RT}*** | ***FSM_{RR}*** | ***FSM_{LT}*** | ***FSM_{LR}*** |
|---|---|---|---|---|
| Pair of pads | Right Translation | Right Rotation | Left Translation | Left Rotation |
| Motion of flexible elongated medical element | | | | |
| State variable | State_{RT} | State_{RR} | State_{LT} | State_{LR} |
| State duration | T_{RT} | T_{RR} | T_{LT} | T_{LR} |
| Position | X_{R} | Z_{R} | X_{L} | Z_{L} |
| Clamping | Y_{R} | | Y_{L} | |

The state of each FSM seems to be defined by what would appear to be "3 ½" variables. In reality, these variables are indeed:
➢ The state; until here, we have described two states "active" and "U-turn" in a first approximation. As explained below, there are indeed more states within a whole cycle,
➢ The duration since the current state is entered; depending of the embodiment, this variable may be or may not be used.
➢ The position of one pad along the x or z axis, depending on which motion the FSM controls,
➢ The position of one pad along the y axis, which controls the clamping status (clamped/unclamped). This is the "half variable" because it is shared between two FSM. For example, the clamping status of the left hand (y_{L}) 15 is related to both FSM_{LT} and FSM_{LR}. This shared variable is a consequence of the fact that unclamping a pair of pads unclamps both the motion along the x and z axis, i.e. stops the translation and the rotation of the flexible elongated medical element 10. In case of disagreement between two FSM regarding y, the priority is given to the unclamping command over the clamping command.

Each FSM changes every Δt. Δt is chosen according to the wanted reactivity of the system and to the actuators characteristics. In a preferred embodiment, Δt = 1 ms.

V_{T} is called the user translation speed setpoint and V_{R} the user rotation speed setpoint (In V_{R}, "R" means "rotation". In x_{R}, y_{R} and z_{R}, it means "right").

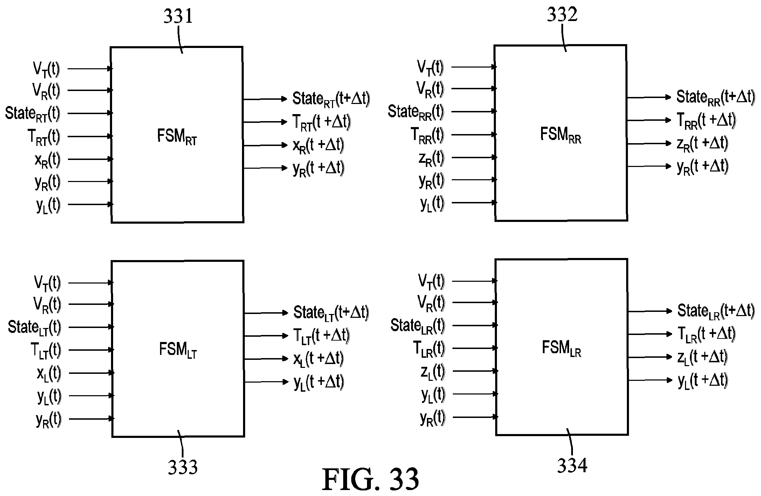

Fig. 33 shows schematically the temporal evolution of four finite state machines, in an example of a catheter robot module according to the invention.

The temporal evolution of the four FSM 331, 332, 333 and 334, is described in the figure 33.

In finite state machine (FSM) 331, dedicated to right translation, i.e. translation of pair 16 of pads 13 and 14:
➢ Inputs are (top-down) at time t:
   ∘ User translation speed setpoint V_{T}(t),
   ∘ User rotation speed setpoint V_{R}(t),
   ∘ Type of state, for translation of pair 16 of pads 13 and 14, State_{RT}(t),
   ∘ Duration of said state, for translation of pair 16 of pads 13 and 14, T_{RT}(t),
   ∘x position, for pair 16 of pads 13 and 14, X_{R}(t),
   ∘ clamping, for pair 16 of pads 13 and 14, Y_{R}(t),
   ∘ clamping, for pair 15 of pads 11 and 12, Y_{L}(t),
Outputs are (top-down) at time t+ Δt:
   ∘ Type of state, for translation of pair 16 of pads 13 and 14, State_{RT}(t+Δt),
   ∘ Duration of said state, for right translation, T_{RT}(t+Δt),
   ∘ x position, for pair 16 of pads 13 and 14, X_{R}(t+Δt),
   ∘ clamping, for pair 16 of pads 13 and 14, Y_{R}(t+Δt).

In finite state machine (FSM) 332, dedicated to right rotation, i.e. rotation of pair 16 of pads 13 and 14:
➢ Inputs are (top-down) at time t:
   ∘ User translation speed setpoint V_{T}(t),
   ∘ User rotation speed setpoint V_{R}(t),
   ∘ Type of state, for rotation of pair 16 of pads 13 and 14, State_{RR}(t),
   ∘ Duration of said state, for rotation of pair 16 of pads 13 and 14, T_{RR}(t),
   ∘ z position, for pair 16 of pads 13 and 14, Z_{R}(t),
   ∘ clamping, for pair 16 of pads 13 and 14, Y_{R}(t),
   ∘ clamping, for pair 15 of pads 11 and 12, Y_{L}(t),
Outputs are (top-down) at time t+ Δt:
   ∘ type of state, for rotation of pair 16 of pads 13 and 14, State_{RR}(t+Δt),
   ∘ Duration of said state, for rotation of pair 16 of pads 13 and 14, T_{RR}(t+Δt),
   ∘ z position, for pair 16 of pads 13 and 14, Z_{R}(t+Δt),
   ∘ clamping, for pair 16 of pads 13 and 14, Y_{R}(t+Δt).

In finite state machine (FSM) 333, dedicated to left translation, i.e. translation of pair 15 of pads 11 and 12:
➢ Inputs are (top-down) at time t:
   ∘ User translation speed setpoint V_{T}(t),
   ∘ User rotation speed setpoint V_{R}(t),
   ∘ Type of state, for translation of pair 15 of pads 11 and 12, State_{LT}(t),
   ∘ Duration of said state, for translation of pair 15 of pads 11 and 12, T_{LT}(t),
   ∘ x position, for pair 15 of pads 11 and 12, X_{L}(t),
   ∘ clamping, for pair 15 of pads 11 and 12, Y_{L}(t),
   ∘ clamping, for pair 16 of pads 13 and 14, Y_{R}(t),
Outputs are (top-down) at time t+ Δt:
   ∘ Type of state, for translation of pair 15 of pads 11 and 12, State_{LT}(t+Δt),
   ∘ Duration of said state, for right translation, T_{LT}(t+Δt),
   ∘ x position, for pair 15 of pads 11 and 12, X_{L}(t+Δt),
   ∘ clamping, for pair 15 of pads 11 and 12, Y_{L}(t+Δt).

In finite state machine (FSM) 334, dedicated to left rotation, i.e. rotation of pair 15 of pads 11 and 12:
➢ Inputs are (top-down) at time t:
   ∘ User translation speed setpoint V_{T}(t),
   ∘ User rotation speed setpoint V_{R}(t),
   ∘ Type of state, for rotation of pair 15 of pads 11 and 12, State_{LR}(t),
   ∘ Duration of said state, for rotation of pair 15 of pads 11 and 12, T_{LR}(t),
   ∘ z position, for pair 15 of pads 11 and 12, Z_{L}(t),
   ∘ clamping, for pair 15 of pads 11 and 12, Y_{L}(t),
   ∘ clamping, for pair 16 of pads 13 and 14, Y_{R}(t),
Outputs are (top-down) at time t+ Δt:
   ∘ type of state, for rotation of pair 15 of pads 11 and 12, State_{LR}(t+Δt),
   ∘ Duration of said state, for rotation of pair 15 of pads 11 and 12, T_{LR}(t+Δt),
   ∘ z position, for pair 15 of pads 11 and 12, Z_{L}(t+Δt),
   ∘ clamping, for pair 15 of pads 11 and 12, Y_{L}(t+Δt).

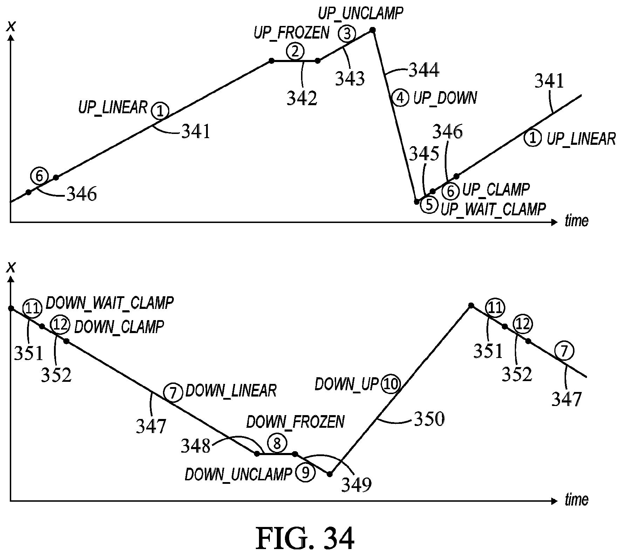

Fig. 34 shows schematically the 12 states of a cycle of the four finite state machines, in an example of a catheter robot module according to the invention.

All the states for FSM_{RT} 331 (i.e. the possible values of State_{RT}) are now described. Man skilled in the art will easily transpose for the 3 other FSM 332, 333 and 334.

There is a total of 12 states. These 12 states are split into two groups: the states 1 to 6 correspond to a positive user setpoint translation speed and the states 7 to 12 to a negative user setpoint translation speed:
➢ state 341 UP_LINEAR: this is the active phase.
➢ state 342 UP_FROZEN: the motion of the pads, and thus the flexible elongated medical element, is stopped.
➢ state 343 UP_UNCLAMP: continue to impart a linear motion along the x axis according to the user speed setpoint, while sending an "unclamp" command the pads along the y axis.
➢ state 344 UP_DOWN: this the U-turn phase.
➢ state 345 UP_WAIT_CLAMP: during this state, the speed of the pair of pads along the x axis should be equal to the user translation speed setpoint, as in the 1 - UP_LINEAR phase.
➢ state 346 UP_CLAMP: during this state, the pads are re-clamped while maintaining the speed of the pads along the x axis equal to the user translation speed setpoint.
➢ state 347 DOWN_LINEAR: this is the active phase.
➢ state 348 DOWN_FROZEN: the motion of the pads, and thus the flexible elongated medical element, is stopped.
➢ state 349 DOWN_UNCLAMP: continue to impart a linear motion along the x axis according to the user speed setpoint, while sending an "unclamp" command the pads along the y axis.
➢ state 350 DOWN_UP: this the U-turn phase.
➢ state 351 DOWN_WAIT_CLAMP: during this state, the target speed of the pair of pads along the x axis should be equal to the user translation speed setpoint, as in the 7 - DOWN_LINEAR phase.
➢ state 352 DOWN_CLAMP: during this state, the pads are re-clamped while maintaining the speed of the pads along the x axis equal to the user translation speed setpoint.

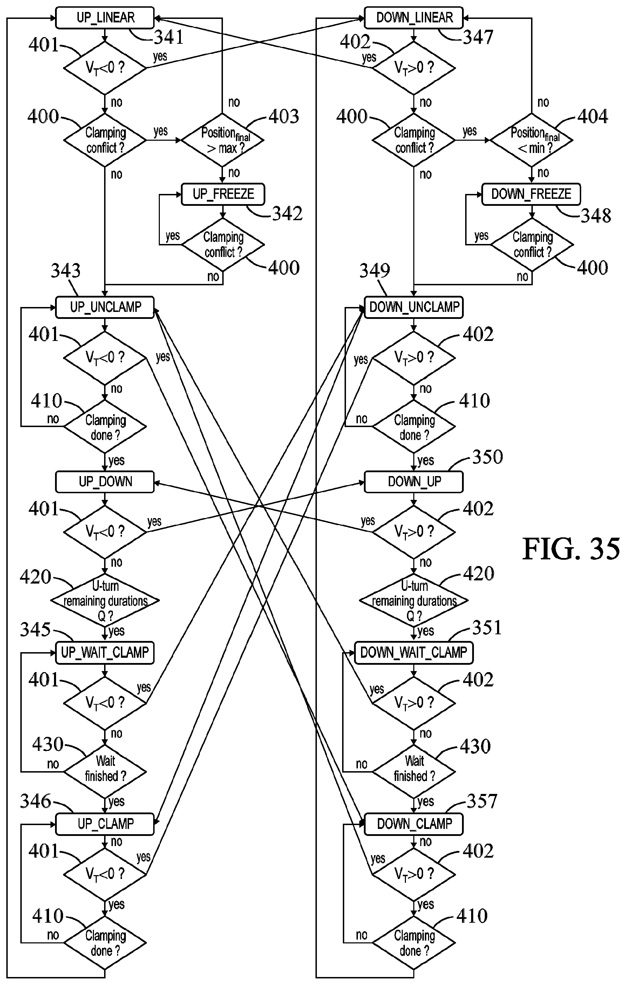

Fig. 35 shows schematically a synoptic representing a cycle of the four finite state machines 331, 332, 333 and 334, in an example of a catheter robot module according to embodiments of the invention.

The state 347 is the equivalent of state 341 in case of a negative value, etc... Therefore, there is a detailed explanation for the states 341 to 346 which is similar to the one given for states 347 to 352.

States 341 to 352 are:
➢ state 341 UP_LINEAR: this is the active phase. The target speed of the pair of pads along the x axis is equal to the user translation speed setpoint. The margin mechanism is active. This mechanism uses, as input data, the final position of the next phase 343 (UP_UNCLAMP), if switching to this phase now. Let's call this position_{final} 403. If position_{final} 403 has reached the maximum value of the standard range, then switch to the UP_UNCLAMP state 343, if the other (left) hand is clamped. If the other hand is not clamped, remain in the UP_LINEAR state 341 until position_{final} reaches the maximum value of the maximum range. If the other hand is still unclamped at this point 400, then switch to UP_FROZEN state 342. This case is an emergency measure in case the margin has not been enough to solve a clamping conflict: a well-designed system should never enter in this state and go directly to UP_UNCLAMP state 343 instead.

The pads should normally be clamped during the UP_LINEAR phase 341. However, since the clamping status is shared between translation and rotation, FSM_{RR} may have decided to unclamp 400 the pads. During this unclamping time, the motion of the pads along the x axis (for translation) will continue normally, although it will have of course no effect on the flexible elongated medical element: the translation will then be obtained thanks to other pair of pads.

If the user translation speed setting becomes negative 401 during this phase, then switch to DOWN_LINEAR 347.
➢ state 342 UP_FROZEN: the motion of the pads, and thus the flexible elongated medical element, is stopped. As soon as the other hand is clamped again 400, switch to UP_UNCLAMP 343.

In this degraded mode, the wanted motion cannot be imparted to the flexible elongated medical element. However, we still clamp it, in order to ensure, at least, that the flexible elongated medical element handled by the system and cannot move freely in the patient, which would be dangerous.

If the user translation speed setting becomes negative 401 during this phase, then switch to phase DOWN_LINEAR 347. There is no switching to DOWN_FROZEN 348 because, in this case, the situation is different: the position is close to the maximum and the position is to be diminished. Therefore, there is no need to unclamp and no more conflict.
➢ state 343 UP_UNCLAMP: continue to impart a linear motion along the x axis according to the user speed setpoint, while sending an "unclamp" command the pads along the y axis. If the pads were already unclamped due to FSM_{RR}, then nothing has to be done. In a preferred embodiment, the UP_UNCLAMP duration is fixed. This makes the calculation of position_{final} during the UP_LINEAR state 341 possible. In another preferred embodiment, the UP_UNCLAMP travel range is fixed. Those two embodiments are interesting but other embodiments can be put in place as long as the calculation is possible during the UP_LINEAR state 341 (earlier in time).

When the UP_UNCLAMP state 343 is finished 410, switch to UP_DOWN 344.

If the user translation speed setting becomes negative during this phase 401, then switch to phase DOWN_CLAMP 352.
➢ state 344 UP_DOWN: this the U-turn phase. The pads have to be unclamped during this phase. The "U-turn duration adaptation mechanism" is active during this phase, in order to determine its duration. This mechanism must be active so as to refresh constantly the duration, which can vary according to the user speed setpoints.

When the UP_DOWN state 346 is finished 420 (the calculated remaining duration is zero or negative) then switch to UP_WAIT_CLAMP 345.

If the user translation speed setting becomes negative during this phase 401, then switch to DOWN_UP 350.
➢ state 345 UP_WAIT_CLAMP: during this state, the speed of the pair of pads along the x axis should be equal to the user translation speed setpoint, as in the 341 UP_LINEAR phase. However, due to the actuators and the mechanics, the system has a response time to switch from the UP_DOWN 344 to the UP_LINEAR 341 speed. Therefore, during this phase, the actuators receive control signals to obtain a linear motion at the UP_LINEAR speed, but the pads have not reached that speed yet. At this stage, it is thus too early to re-clamp: the pads have to remain unclamped.

In a preferred embodiment, the UP_WAIT_CLAMP duration is fixed. In another preferred embodiment, the UP_WAIT_CLAMP travel range is fixed. In a third preferred embodiment, the duration is variable, and the state can be quit when the actual speed of the pads is close enough to the wanted speed. In a preferred embodiment, "enough" could be defined as the absolute value of the difference in percentage being lower than a predetermined threshold.

When the state is finished 430, according to one of the criteria explained above, switch to UP_CLAMP 346.

If the user translation speed setting becomes negative during this phase 401, then switch to DOWN_UNCLAMP 349.
➢ state 346 UP_CLAMP: during this state, the pads are re-clamped while maintaining the speed of the pads along the x axis equal to the user translation speed setpoint.

In a preferred embodiment, the UP_CLAMP duration is fixed. In another preferred embodiment, the UP_CLAMP travel range is fixed. In a third preferred embodiment, the duration is variable, and the state can be quit when the position of the pads along the y axis has reached a predetermined value. In a fourth preferred embodiment the duration is variable, and the state can be quit when the push force applied to the pads along the y axis has reached a predetermined value.

When the state is finished 410, according to one of the criteria explained above, switch to UP_LINEAR 341.

If the user translation speed setting becomes negative during this phase 401, then switch to DOWN_UNCLAMP 349.
➢ state 347 DOWN_LINEAR: this is the active phase. The target speed of the pair of pads along the x axis is equal to the user translation speed setpoint. The margin mechanism is active. This mechanism uses, as input data, the final position of the next phase 349 (DOWN_UNCLAMP), if switching to this phase now. Let's call this position_{final} 404. If position_{final} 404 has reached the minimum value of the standard range, then switch to the DOWN_UNCLAMP state 349, if the other (left) hand is clamped. If the other hand is not clamped, remain in the DOWN_LINEAR state 347 until position_{final} reaches the minimum value of the maximum range. If the other hand is still unclamped at this point 400, then switch to DOWN_FROZEN state 348. This case is an emergency measure in case the margin has not been enough to solve a clamping conflict: a well-designed system should never enter in this state and go directly to DOWN_UNCLAMP state 349 instead.

The pads should normally be clamped during the DOWN_LINEAR phase 347. However, since the clamping status is shared between translation and rotation, FSM_{RR} may have decided to unclamp 400 the pads. During this unclamping time, the motion of the pads along the x axis (for translation) will continue normally, although it will have of course no effect on the flexible elongated medical element: the translation will then be obtained thanks to other pair of pads.

If the user translation speed setting becomes positive 402 during this phase, then switch to UP_LINEAR 341.
➢ state 348 DOWN_FROZEN: the motion of the pads, and thus the flexible elongated medical element, is stopped. As soon as the other hand is clamped again 400, switch to DOWN_UNCLAMP 349.

In this degraded mode, the wanted motion cannot be imparted to the flexible elongated medical element. However, we still clamp it, in order to ensure, at least, that the flexible elongated medical element handled by the system and cannot move freely in the patient, which would be dangerous.

If the user translation speed setting becomes positive 402 during this phase, then switch to phase UP_LINEAR 341. There is no switching to UP_FROZEN 342 because, in this case, the situation is different: the position is close to the minimum and the position is to be increased. Therefore, there is no need to unclamp and no more conflict.
➢ state 349 DOWN_UNCLAMP: continue to impart a linear motion along the x axis according to the user speed setpoint, while sending an "unclamp" command the pads along the y axis. If the pads were already unclamped due to FSM_{RR}, then nothing has to be done. In a preferred embodiment, the DOWN_UNCLAMP duration is fixed. This makes the calculation of position_{final} during the DOWN_LINEAR state 347 possible. In another preferred embodiment, the DOWN_UNCLAMP travel range is fixed. Those two embodiments are interesting but other embodiments can be put in place as long as the calculation is possible during the DOWN_LINEAR state 347 (earlier in time).

When the DOWN_UNCLAMP state 349 is finished 410, switch to DOWN_UP 350.

If the user translation speed setting becomes positive during this phase 402, then switch to phase UP_CLAMP 356.
➢ state 350 DOWN_UP: this the U-turn phase. The pads have to be unclamped during this phase. The "U-turn duration adaptation mechanism" is active during this phase, in order to determine its duration. This mechanism must be active so as to refresh constantly the duration, which can vary according to the user speed setpoints.

When the DOWN_UP state 350 is finished 420 (the calculated remaining duration is zero or negative) then switch to DOWN_WAIT_CLAMP 351.

If the user translation speed setting becomes positive during this phase 402, then switch to UP_DOWN 344.
➢ state 351 DOWN_WAIT_CLAMP: during this state, the target speed of the pair of pads along the x axis should be equal to the user translation speed setpoint, as in the 347 DOWN_LINEAR phase. However, due to the actuators and the mechanics, the system has a response time to switch from the DOWN_UP 350 to the DOWN_LINEAR 347 speed. Therefore, during this phase, the actuators receive control signals to obtain a linear motion at the DOWN_LINEAR speed, but the pads have not reached that speed yet. At this stage, it is thus too early to re-clamp: the pads have to remain unclamped.

In a preferred embodiment, the DOWN_WAIT_CLAMP duration is fixed. In another preferred embodiment, the DOWN _ WAIT CLAMP travel range is fixed. In a third preferred embodiment, the duration is variable, and the state can be quit when the actual speed of the pads is close enough to the wanted speed. In a preferred embodiment, "enough" could be defined as the absolute value of the difference in percentage being lower than a predetermined threshold.

When the state is finished 430, according to one of the criteria explained above, switch to DOWN_CLAMP 352.

If the user translation speed setting becomes positive during this phase 402, then switch to UP_UNCLAMP 343.
➢ state 352 DOWN_CLAMP: during this state, the pads are re-clamped while maintaining the speed of the pads along the x axis equal to the user translation speed setpoint.

In a preferred embodiment, the DOWN_CLAMP duration is fixed. In another preferred embodiment, the DOWN_CLAMP travel range is fixed. In a third preferred embodiment, the duration is variable, and the state can be quit when the position of the pads along the y axis has reached a predetermined value. In a fourth preferred embodiment the duration is variable, and the state can be quit when the push force applied to the pads along the y axis has reached a predetermined value.

When the state is finished 410, according to one of the criteria explained above, switch to DOWN_LINEAR 347.

If the user translation speed setting becomes positive during this phase 402, then switch to UP_UNCLAMP 343.

An additional state is needed, which is not shown on figure 35 which is the initialization state. In a preferred embodiment, it will initialize all its variable and branch to the UP_LINEAR state 341. In a preferred embodiment, the position of the left hand 15 along the x axis is at ¼ of the standard range, while the position of the right hand 16 is at ¾ of the standard range. The same applies to the z axis. Regarding the y axis, both hands are clamped.

When the translation speed setpoint (V_{T}) changes, there is a jump from the UP_ to the DOWN_states and vice-versa. If the value goes to 0, however, the state does not change. When V_{T}=0, the state machine stops where it is, except for the UP_DOWN and DOWN_UP state, which continue until their end.

The invention has been described with reference to preferred embodiments. However, many variations are possible within the scope of the invention.

## Claims

1. Catheter robot module for translation and rotation of a flexible elongated medical element (10), comprising:
➢ a casing,
➢ two pairs (15, 16) of movable pads (11, 12, 13, 14):
∘ said pads of a same pair at least partly facing each other,
∘ each pair of movable pads being adapted to separately or in combination:
➢ perform a translation of said flexible elongated medical element longitudinally with respect to said casing, by a first translation cycle:
• clamping said flexible elongated medical element between said pads,
• translating forth said pads synchronously longitudinally in the same direction with respect to said casing, with respect to a user set longitudinal translation direction,
• unclamping said flexible elongated medical element,
• translating back said pads synchronously longitudinally in the same reverse direction with respect to said casing, with respect to said user set longitudinal translation direction,
▪ perform a rotation of said flexible elongated medical element around longitudinal axis with respect to said casing, by a second rotation cycle:
• clamping said flexible elongated medical element between said pads,
• performing a relative forth translation of said pads transversely in opposite directions with respect to said casing, with respect to a transversal translation direction corresponding to a set rotation direction,
• unclamping said flexible elongated medical element,
• performing a relative back translation of said pads transversely in opposite reverse directions with respect to said casing, with respect to said transversal translation direction corresponding to said set rotation direction,
➢ a driver of said pairs of movable pads implemented so that:
∘ in at least one mode where, in combination, said translation of said flexible elongated medical element is alternatively performed by said pairs of movable pads, both said pairs working in phase opposition, and said rotation of said flexible elongated medical element is performed by at least one of said pairs of movable pads,
▪ **characterized in that** conflict of synchronization between said translation and said rotation is managed at least:
• by varying travel extension of said forth translation in said first translation cycle for at least one of said pairs, and/or by varying travel extension and/or duration of said forth translation in said second rotation cycle for at least one of said pairs,
∘ so as to always keep at least one pair of movable pads clamped on said flexible elongated medical element, during the whole duration of said translation of said flexible elongated medical element in said first translation cycle as well as during the whole duration of said rotation of said flexible elongated medical element in said second rotation cycle.

2. Catheter robot module according to claim 1, wherein:
➢ said driver of said pairs of movable pads is implemented so that:
∘ in one or several or all modes where said translation of said flexible elongated medical element is performed:
• said translation of said flexible elongated medical element is alternatively performed by said pairs of movable pads, both said pairs working in phase opposition, said phase opposition being controlled at least:
• by varying duration of said translating back in said first translation cycle for at least one of said pairs,
∘ so as to control and keep said phase opposition between both said pairs.

3. Catheter robot module according to any of preceding claims, wherein said rotation of said flexible elongated medical element (10) is alternatively performed by said pairs (15, 16) of movable pads (11, 12, 13, 14),
and wherein preferably:
➢ said rotation of said flexible elongated medical element (10) is alternatively performed by said pairs (15, 16) of movable pads (11, 12, 13, 14), both said pairs working in phase opposition, said phase opposition being controlled, at least:
∘ by varying duration of said translating back in said second rotation cycle for at least one of said pairs,
• so as to control and keep said phase opposition between both said pairs.

4. Catheter robot module according to any of preceding claims, wherein:
➢ said driver of said pairs of movable pads implemented so that:
∘ said translation of said flexible elongated medical element being alternatively performed by said pairs of movable pads, both said pairs working in phase opposition,
∘ said rotation of said flexible elongated medical element being alternatively performed by said pairs of movable pads,
∘ conflict of synchronization between said translation and said rotation being managed, at least:
• by varying travel extension and/or duration of said forth translation, in said first translation cycle and/or in said second rotation cycle, for both of said pairs,
• so as to always keep at least one pair of movable pads clamped on said flexible elongated medical element, during the whole duration of said translation of said flexible elongated medical element in said first translation cycle as well as during the whole duration of said rotation of said flexible elongated medical element in said second rotation cycle.

5. Catheter robot module according to claim 4, wherein:
➢ said driver of said pairs of movable pads is implemented so that:
∘ said phase opposition is controlled, at least:
• by varying duration of said translating back, in said first translation cycle and in said second rotation cycle, for at least one of said pairs,
• so as to control and keep said phase opposition between both said pairs.

6. Catheter robot module according to any of preceding claims, wherein:
➢ said driver of said pairs (15, 16) of movable pads (11, 12, 13, 14) is implemented so that:
∘ said translation of said flexible elongated medical element (10) is alternatively performed by said pairs of movable pads, both pairs working in phase opposition, said phase opposition being controlled mainly or only:
• by varying duration of said translating back, in said first translation cycle for at least one of said pairs,
• so as to control and keep said phase opposition between both said pairs.

7. Catheter robot module according to any of preceding claims, wherein:
➢ said driver of said pairs (15, 16) of movable pads (11, 12, 13, 14) is implemented so that:
∘ said rotation of said flexible elongated medical element (10) is alternatively performed by said pairs of movable pads,
∘ conflict of synchronization between said translation and said rotation is managed mainly or only:
• by varying travel extension of said forth translation, in said first translation cycle for at least one of said pairs,
• so as to always keep at least one pair of movable pads clamped on said flexible elongated medical element, during the whole duration of said translation of said flexible elongated medical element in said first cycle as well as during the whole duration of said rotation of said flexible elongated medical element in said second cycle.

8. Catheter robot module according to any of preceding claims, wherein said forth translation duration is always longer than said back translation duration.

9. Catheter robot module according to any of preceding claims, wherein said varying travel extension of said forth translation in said first translation cycle for one of said pairs is performed by extending a predetermined standard forth translation travel range, reaching a value ranging from said predetermined standard forth translation travel range to a predetermined maximum forth translation travel range,
and wherein preferably:
said predetermined maximum forth translation travel range is comprised between 110% and 150% of said predetermined standard forth translation travel range, preferably between 120% and 140% of said predetermined standard forth translation travel range,
and/or wherein preferably
said predetermined maximum forth translation travel range is split in two equal parts respectively at both ends of said predetermined standard forth translation travel range.

10. Catheter robot module according to any of preceding claims, wherein there is some temporary overlapping between said flexible elongated medical element (10) clamping by one of said pairs of movables pads and said flexible elongated medical element clamping by the other one of said pairs of movables pads, said temporary overlapping lasting preferably between 10% and 95% of the whole duration of said translation of said flexible elongated medical element.

11. Catheter robot module according to any of preceding claims, wherein said flexible elongated medical element (10) unclamping is performed simultaneously to a portion of said forth translation travel extension, during the second half of said forth translation travel extension, said portion ranging preferably from 5% to 20% of the full extent of said forth translation travel extension.

12. Catheter robot module according to any of preceding claims, wherein said flexible elongated medical element (10) clamping is performed simultaneously to a portion of said forth translation travel extension, during the first half of said forth translation travel extension, said portion ranging preferably from 5% to 20% of the full extent of said forth translation travel extension.

13. Catheter robot module according to any of preceding claims, wherein said flexible elongated medical element (10) clamping starts after the end of said back translation travel extension and after the beginning of next said forth translation travel extension.

14. Catheter robot module according to any of preceding claims, wherein said varying duration of said translating back in said first translation cycle for one of said pairs (15, 16), so as to control and keep said phase opposition between both said pairs (15, 16), is performed by reducing or extending duration with respect to a standard back translation duration,
and wherein preferably:
said varying duration of said translating back in said first translation cycle for one of said pairs (15, 16), so as to control and keep said phase opposition between both said pairs (15, 16), is performed by reducing or extending duration with respect to a standard back translation duration less than requested for optimal phase opposition controlling and keeping so as to improve stability to the cost of higher number of cycles to get back at phase opposition target, a factor α of correction attenuation comprised between 0 and 1 being applied,
and/or wherein preferably:
said factor α of correction attenuation is comprised between 0.3 and 0.7, and is preferably about 0.5.

15. Catheter robot module according to any of preceding claims, wherein said standard back translation duration is a decreasing function of a user command speed target value(s), for either translation and/or rotation, preferably minimum of both speed target values, when applicable, becoming selected user command speed target value,
and wherein preferably:
said decreasing function presents a central curved part (280) which presents a concavity toward top and which is located between two horizontal parts (281, 282),
and/or wherein preferably:
said central curved part (280) is inversely proportional to said selected user command speed target value, whereas said horizontal parts (281, 282) are constant with respect to said selected user command speed target value.

16. Catheter robot module according to any of preceding claims, wherein:
➢ said user set longitudinal translation direction can be varied continuously by said user,
➢ and/or said user set rotation direction can be varied continuously by said user.

17. Catheter robot module according to any of preceding claims, wherein:
➢ said translation of said flexible elongated medical element (10) longitudinally for first (16) of said pairs of pads (13, 14) is performed by a first finite state machine (331),
➢ said rotation of said flexible elongated medical element around longitudinal axis with respect to said casing for first (16) of said pairs of pads (13, 14) is performed by a second finite state machine (332),
➢ said translation of said flexible elongated medical element longitudinally for second (15) of said pairs of pads (11, 12) is performed by a third finite state machine (333),
➢ said rotation of said flexible elongated medical element around longitudinal axis with respect to said casing for second (15) of said pairs of pads (11, 12) is performed by a fourth finite state machine (334),
and wherein preferably:
➢ each of said finite state machines determines for a transition period between said forth translation and said back translation to go progressively from said forth translation to said back translation:
∘ start of said transition period,
∘ duration of said transition period,
∘ end of said transition period,
and/or wherein preferably:
each of said finite state machines changes periodically with a period (Δt) which is less than 5ms, preferably comprised between 0.5ms and 2ms, more preferably about 1ms.

## Patentansprüche

1. **Katheterrobotermodul** zur Translation und Rotation eines flexiblen länglichen medizinischen Elements (10), umfassend:
> ein Gehäuse,
> zwei Paare (15, 16) beweglicher Pads (11, 12, 13, 14):
∘ wobei die Pads eines gleichen Paares einander wenigstens teilweise zugewandt sind,
∘ wobei jedes Paar beweglicher Pads dazu eingerichtet ist, separat oder in Kombination:
▪ eine Translation des flexiblen länglichen medizinischen Elements in Längsrichtung in Bezug auf das Gehäuse durchzuführen, durch einen ersten Translationszyklus:
• Klemmen des flexiblen länglichen medizinischen Elements zwischen den Pads,
• synchrone Vorwärtstranslation der Pads in Längsrichtung in der gleichen Richtung in Bezug auf das Gehäuse, in Bezug auf eine von einem Benutzer festgelegte Längstranslationsrichtung,
• Lösen des flexiblen länglichen medizinischen Elements,
• synchrone Rückwärtstranslation der Pads in Längsrichtung in der gleichen entgegengesetzten Richtung in Bezug auf das Gehäuse, in Bezug auf die von einem Benutzer festgelegte Längstranslationsrichtung,
▪ eine Rotation des flexiblen länglichen medizinischen Elements um eine Längsachse in Bezug auf das Gehäuse durchzuführen, durch einen zweiten Rotationszyklus:
• Klemmen des flexiblen länglichen medizinischen Elements zwischen den Pads,
• **Durchführen** einer relativen Vorwärtstranslation der Pads transversal in entgegengesetzten Richtungen in Bezug auf das Gehäuse, in Bezug auf eine transversale Translationsrichtung, welche einer festgelegten Rotationsrichtung entspricht,
• Lösen des flexiblen länglichen medizinischen Elements,
• Durchführen einer relativen Rückwärtstranslation der Pads transversal in entgegengesetzten Gegenrichtungen in Bezug auf das Gehäuse, in Bezug auf die transversale Translationsrichtung, welche der festgelegten Rotationsrichtung entspricht,
➢ eine Antriebseinheit der Paare beweglicher Pads, welche so implementiert ist, dass:
∘ in wenigstens einem Modus, in welchem, in Kombination, die Translation des flexiblen länglichen medizinischen Elements abwechselnd von den Paaren beweglicher Pads durchgeführt wird, wobei beide Paare in Phasenopposition arbeiten, und die Rotation des flexiblen länglichen medizinischen Elements von wenigstens einem der Paare beweglicher Pads durchgeführt wird,
▪ **dadurch gekennzeichnet, dass** ein Synchronisationskonflikt zwischen der Translation und der Rotation wenigstens verwaltet wird:
• durch Variieren eines Verfahrwegs der Vorwärtstranslation in dem ersten Translationszyklus für wenigstens eines der Paare, und/oder durch Variieren eines Verfahrwegs und/oder einer Dauer der Vorwärtstranslation in dem zweiten Rotationszyklus für wenigstens eines der Paare,
∘ derart, dass stets wenigstens ein Paar beweglicher Pads auf dem flexiblen länglichen medizinischen Element geklemmt bleibt, während der gesamten Dauer der Translation des flexiblen länglichen medizinischen Elements in dem ersten Translationszyklus sowie während der gesamten Dauer der Rotation des flexiblen länglichen medizinischen Elements in dem zweiten Rotationszyklus.

2. Katheterrobotermodul nach Anspruch 1, wobei:
> die Antriebseinheit der Paare beweglicher Pads so implementiert ist, dass:
∘in einem oder mehreren oder allen Modi, in welchen die Translation des flexiblen länglichen medizinischen Elements durchgeführt wird:
▪ die Translation des flexiblen länglichen medizinischen Elements abwechselnd von den Paaren beweglicher Pads durchgeführt wird, wobei beide Paare in Phasenopposition arbeiten, wobei die Phasenopposition wenigstens gesteuert wird:
• durch Variieren einer Dauer der Rückwärtstranslation in dem ersten Translationszyklus für wenigstens eines der Paare,
∘ derart, dass die Phasenopposition zwischen beiden Paaren gesteuert und aufrechterhalten wird.

3. Katheterrobotermodul nach einem der vorhergehenden Ansprüche, wobei die Rotation des flexiblen länglichen medizinischen Elements (10) abwechselnd von den Paaren (15, 16) beweglicher Pads (11, 12, 13, 14) durchgeführt wird,
und wobei vorzugsweise:
➢ die Rotation des flexiblen länglichen medizinischen Elements (10) abwechselnd von den Paaren (15, 16) beweglicher Pads (11, 12, 13, 14) durchgeführt wird, wobei beide Paare in Phasenopposition arbeiten, wobei die Phasenopposition wenigstens gesteuert wird:
∘ durch Variieren einer Dauer der Rückwärtstranslation in dem zweiten Rotationszyklus für wenigstens eines der Paare,
▪ derart, dass die Phasenopposition zwischen beiden Paaren gesteuert und aufrechterhalten wird.

4. Katheterrobotermodul nach einem der vorhergehenden Ansprüche, wobei:
➢ die Antriebseinheit der Paare beweglicher Pads so implementiert ist, dass:
∘ die Translation des flexiblen länglichen medizinischen Elements abwechselnd von den Paaren beweglicher Pads durchgeführt wird, wobei beide Paare in Phasenopposition arbeiten,
∘ die Rotation des flexiblen länglichen medizinischen Elements abwechselnd von den Paaren beweglicher Pads durchgeführt wird,
∘ ein Synchronisationskonflikt zwischen der Translation und der Rotation wenigstens verwaltet wird:
▪ durch Variieren eines Verfahrwegs und/oder einer Dauer der Vorwärtstranslation, in dem ersten Translationszyklus und/oder in dem zweiten Rotationszyklus, für beide Paare,
• derart, dass stets wenigstens ein Paar beweglicher Pads auf dem flexiblen länglichen medizinischen Element geklemmt bleibt, während der gesamten Dauer der Translation des flexiblen länglichen medizinischen Elements in dem ersten Translationszyklus sowie während der gesamten Dauer der Rotation des flexiblen länglichen medizinischen Elements in dem zweiten **Rotationszyklus.**

5. Katheterrobotermodul nach Anspruch 4, wobei:
> die Antriebseinheit der Paare beweglicher Pads so implementiert ist, dass:
∘ die Phasenopposition wenigstens gesteuert wird:
▪ durch Variieren einer Dauer der Rückwärtstranslation, in dem ersten Translationszyklus und in dem zweiten Rotationszyklus, für wenigstens eines der Paare,
• derart, dass die Phasenopposition zwischen beiden Paaren gesteuert und aufrechterhalten wird.

6. Katheterrobotermodul nach einem der vorhergehenden Ansprüche, wobei:
> die Antriebseinheit der Paare (15, 16) beweglicher Pads (11, 12, 13, 14) so implementiert ist, dass:
∘ die Translation des flexiblen länglichen medizinischen Elements (10) abwechselnd von den Paaren beweglicher Pads durchgeführt wird, wobei beide Paare in Phasenopposition arbeiten, wobei die Phasenopposition hauptsächlich oder ausschließlich gesteuert wird:
▪ durch Variieren einer Dauer der Rückwärtstranslation, in dem ersten Translationszyklus für wenigstens eines der Paare,
• derart, dass die Phasenopposition zwischen beiden Paaren gesteuert und aufrechterhalten wird.

7. Katheterrobotermodul nach einem der vorhergehenden Ansprüche, wobei:
> die Antriebseinheit der Paare (15, 16) beweglicher Pads (11, 12, 13, 14) so implementiert ist, dass:
∘ die Rotation des flexiblen länglichen medizinischen Elements (10) abwechselnd von den Paaren beweglicher Pads durchgeführt wird,
∘ ein Synchronisationskonflikt zwischen der Translation und der Rotation hauptsächlich oder ausschließlich verwaltet wird:
▪ durch Variieren eines Verfahrwegs der Vorwärtstranslation, in dem ersten Translationszyklus für wenigstens eines der Paare,
• derart, dass stets wenigstens ein Paar beweglicher Pads auf dem flexiblen länglichen medizinischen Element geklemmt bleibt, während der gesamten Dauer der Translation des flexiblen länglichen medizinischen Elements in dem ersten Zyklus sowie während der gesamten Dauer der Rotation des flexiblen länglichen medizinischen Elements in dem zweiten Zyklus.

8. Katheterrobotermodul nach einem der vorhergehenden Ansprüche, wobei die Vorwärtstranslationsdauer stets länger ist als die Rückwärtstranslationsdauer.

9. Katheterrobotermodul nach einem der vorhergehenden Ansprüche, wobei das Variieren des Verfahrwegs der Vorwärtstranslation in dem ersten Translationszyklus für eines der Paare durch Verlängern eines vorbestimmten Standard-Vorwärtstranslation-Verfahrbereichs durchgeführt wird, wobei ein Wert erreicht wird, welcher von dem vorbestimmten Standard-Vorwärtstranslation-Verfahrbereich und einem vorbestimmten maximalen Vorwärtstranslation-Verfahrbereich reicht,
und wobei vorzugsweise:
der vorbestimmte maximale Vorwärtstranslation-Verfahrbereich zwischen 110 % und 150 % des vorbestimmten Standard-Vorwärtstranslation-Verfahrbereichs umfasst ist, vorzugsweise zwischen 120 % und 140 % des vorbestimmten Standard-Vorwärtstranslation-Verfahrbereichs,
und/oder wobei vorzugsweise:
der vorbestimmte maximale Vorwärtstranslation-Verfahrbereich in zwei gleiche Teile aufgeteilt ist, welche sich jeweils an beiden Enden des vorbestimmten Standard-Vorwärtstranslation-Verfahrbereichs befinden.

10. Katheterrobotermodul nach einem der vorhergehenden Ansprüche, wobei ein zeitweises Überlappen zwischen dem Klemmen des flexiblen länglichen medizinischen Elements (10) durch eines der Paare beweglicher Pads und dem Klemmen des flexiblen länglichen medizinischen Elements durch das andere der Paare beweglicher Pads besteht, wobei das zeitweise Überlappen vorzugsweise zwischen 10 % und 95 % der gesamten Dauer der Translation des flexiblen länglichen medizinischen Elements andauert.

11. Katheterrobotermodul nach einem der vorhergehenden Ansprüche, wobei das Lösen des flexiblen länglichen medizinischen Elements (10) gleichzeitig mit einem Abschnitt des Verfahrwegs der Vorwärtstranslation durchgeführt wird, während der zweiten Hälfte des Verfahrwegs der Vorwärtstranslation, wobei der Abschnitt vorzugsweise zwischen 5 % und 20 % des gesamten Ausmaßes des Verfahrwegs der Vorwärtstranslation beträgt.

12. Katheterrobotermodul nach einem der vorhergehenden Ansprüche, wobei das Klemmen des flexiblen länglichen medizinischen Elements (10) gleichzeitig mit einem Abschnitt des Verfahrwegs der Vorwärtstranslation durchgeführt wird, während der ersten Hälfte des Verfahrwegs der Vorwärtstranslation, wobei der Abschnitt vorzugsweise zwischen 5 % und 20 % des gesamten Ausmaßes des Verfahrwegs der Vorwärtstranslation beträgt.

13. Katheterrobotermodul nach einem der vorhergehenden Ansprüche, wobei das Klemmen des flexiblen länglichen medizinischen Elements (10) nach dem Ende des Verfahrwegs der Rückwärtstranslation und nach dem Beginn eines nächsten Verfahrwegs der Vorwärtstranslation beginnt.

14. Katheterrobotermodul nach einem der vorhergehenden Ansprüche, wobei das Variieren der Dauer der Rückwärtstranslation in dem ersten Translationszyklus für eines der Paare (15, 16), derart, dass die Phasenopposition zwischen beiden Paaren (15, 16) gesteuert und aufrechterhalten wird, durch Verkürzen oder Verlängern der Dauer in Bezug auf eine Standard-Rückwärtstranslationsdauer durchgeführt wird,
und wobei vorzugsweise:
das Variieren der Dauer der Rückwärtstranslation in dem ersten Translationszyklus für eines der Paare (15, 16), derart, dass die Phasenopposition zwischen beiden Paaren (15, 16) gesteuert und aufrechterhalten wird, durch Verkürzen oder Verlängern der Dauer in Bezug auf eine Standard-Rückwärtstranslationsdauer durchgeführt wird, welche geringer ist als für eine optimale Steuerung und Aufrechterhaltung der Phasenopposition erforderlich, um die Stabilität auf Kosten einer höheren Anzahl von Zyklen zur Rückkehr zum Phasenoppositionsziel zu verbessern, wobei ein Korrekturdämpfungsfaktor α zwischen 0 und 1 angewendet wird,
und/oder wobei vorzugsweise:
der Korrekturdämpfungsfaktor α zwischen 0,3 und 0,7 umfasst ist und vorzugsweise etwa 0,5 beträgt.

15. Katheterrobotermodul nach einem der vorhergehenden Ansprüche, wobei die Standard-Rückwärtstranslationsdauer eine abnehmende Funktion eines Benutzerbefehl-Geschwindigkeitszielwerts oder Benutzerbefehl-Geschwindigkeitszielwerte ist, für Translation und/oder Rotation, wobei vorzugsweise ein Minimum beider Geschwindigkeitszielwerte, sofern anwendbar, als Benutzerbefehl-Geschwindigkeitszielwert ausgewählt wird,
und wobei vorzugsweise:
die abnehmende Funktion einen zentralen gekrümmten Teil (280) darstellt, welcher eine nach oben gerichtete Konkavität aufweist und welcher zwischen zwei horizontalen Teilen (281, 282) angeordnet ist, und/oder wobei vorzugsweise:
der zentrale gekrümmte Teil (280) umgekehrt proportional zu dem ausgewählten Benutzerbefehl-Geschwindigkeitszielwert ist, während die horizontalen Teile (281, 282) in Bezug auf den ausgewählten Benutzerbefehl-Geschwindigkeitszielwert konstant sind.

16. Katheterrobotermodul nach einem der vorhergehenden Ansprüche, wobei:
➢ die von einem Benutzer festgelegte Längstranslationsrichtung durch den Benutzer kontinuierlich variiert werden kann,
➢ und/oder die festgelegte Rotationsrichtung durch den Benutzer kontinuierlich variiert werden kann.

17. Katheterrobotermodul nach einem der vorhergehenden Ansprüche, wobei:
➢ die Translation des flexiblen länglichen medizinischen Elements (10) in Längsrichtung für das erste (16) der Paare von Pads (13, 14) durch einen ersten endlichen Automaten (331) durchgeführt wird,
➢ die Rotation des flexiblen länglichen medizinischen Elements um eine Längsachse in Bezug auf das Gehäuse für das erste (16) der Paare von Pads (13, 14) durch einen zweiten endlichen Automaten (332) durchgeführt wird,
➢ die Translation des flexiblen länglichen medizinischen Elements in Längsrichtung für das zweite (15) der Paare von Pads (11, 12) durch einen dritten endlichen Automaten (333) durchgeführt wird,
➢ die Rotation des flexiblen länglichen medizinischen Elements um eine Längsachse in Bezug auf das Gehäuse für das zweite (15) der Paare von Pads (11, 12) durch einen vierten endlichen Automaten (334) durchgeführt wird,
und wobei vorzugsweise:
➢ jeder der endlichen Automaten für eine Übergangsperiode zwischen der Vorwärtstranslation und der Rückwärtstranslation, um progressiv von der Vorwärtstranslation zur Rückwärtstranslation überzugehen, bestimmt:
∘ einen Beginn der Übergangsperiode,
∘ eine Dauer der Übergangsperiode,
∘ ein Ende der Übergangsperiode,
und/oder wobei vorzugsweise:
jeder der endlichen Automaten periodisch mit einer Periode (Δt) wechselt, welche kleiner als 5 ms ist, vorzugsweise zwischen 0,5 ms und 2 ms umfasst ist, besonders bevorzugt etwa 1 ms beträgt.

## Revendications

1. Module robot de cathéter pour la translation et la rotation d'un élément médical allongé flexible (10), comprenant :
➢ un carter,
➢ deux paires (15, 16) de patins mobiles (11, 12, 13, 14) :
∘ lesdits patins d'une même paire faisant au moins partiellement face l'un à l'autre,
∘ chaque paire de patins mobiles étant adaptée pour séparément ou en combinaison :
▪ réaliser une translation dudit élément médical allongé flexible longitudinalement par rapport audit carter, par un premier cycle de translation :
• le serrage dudit élément médical allongé flexible entre lesdits patins,
• la translation avant desdits patins de manière synchrone longitudinalement dans le même sens par rapport audit carter, par rapport à un sens de translation longitudinale défini par l'utilisateur,
• le desserrage dudit élément médical allongé flexible,
• la translation arrière desdits patins de manière synchrone longitudinalement dans le même sens inverse par rapport audit carter, par rapport audit sens de translation longitudinale défini par l'utilisateur,
▪ réaliser une rotation dudit élément médical allongé flexible autour de l'axe longitudinal par rapport audit carter, par un deuxième cycle de rotation :
• le serrage dudit élément médical allongé flexible entre lesdits patins,
• la réalisation d'une translation avant relative desdits patins transversalement dans des sens opposés par rapport audit carter, par rapport à un sens de translation transversale correspondant à un sens de rotation défini,
• le desserrage dudit élément médical allongé flexible,
• la réalisation d'une translation arrière relative desdits patins transversalement dans des sens inverses opposés par rapport audit carter, par rapport audit sens de translation transversale correspondant audit sens de rotation défini,
➢ un pilote desdites paires de patins mobiles étant mis en œuvre de sorte que :
∘ dans au moins un mode où, en combinaison, ladite translation dudit élément médical allongé flexible est réalisée alternativement par lesdites paires de patins mobiles, les deux dites paires travaillant en opposition de phase, et ladite rotation dudit élément médical allongé flexible est réalisée par au moins une parmi lesdites paires de patins mobiles,
▪ **caractérisé en ce qu'**un conflit de synchronisation entre ladite translation et ladite rotation est géré au moins :
• en faisant varier l'extension de course de ladite translation avant dans ledit premier cycle de translation pour au moins une parmi lesdites paires, et/ou en faisant varier l'extension de course et/ou la durée de ladite translation avant dans ledit deuxième cycle de rotation pour au moins une parmi lesdites paires,
∘ de façon à maintenir toujours au moins une paire de patins mobiles serrés sur ledit élément médical allongé flexible, pendant toute la durée de ladite translation dudit élément médical allongé flexible dans ledit premier cycle de translation ainsi que pendant toute la durée de ladite rotation dudit élément médical allongé flexible dans ledit deuxième cycle de rotation.

2. Module robot de cathéter selon la revendication 1, dans lequel :
➢ ledit pilote desdites paires de patins mobiles est mis en œuvre de sorte que :
∘ dans un ou plusieurs ou tous les modes où ladite translation dudit élément médical allongé flexible est réalisée :
▪ ladite translation dudit élément médical allongé flexible est réalisée alternativement par lesdites paires de patins mobiles, les deux dites paires travaillant en opposition de phase, ladite opposition de phase étant contrôlée au moins :
• en faisant varier la durée de ladite translation arrière dans ledit premier cycle de translation pour au moins une parmi lesdites paires,
∘ de façon à contrôler et maintenir ladite opposition de phase entre les deux dites paires.

3. Module robot de cathéter selon l'une quelconque des revendications précédentes, dans lequel ladite rotation dudit élément médical allongé flexible (10) est réalisée alternativement par lesdites paires (15, 16) de patins mobiles (11, 12, 13, 14),
et dans lequel de préférence :
➢ ladite rotation dudit élément médical allongé flexible (10) est réalisée alternativement par lesdites paires (15, 16) de patins mobiles (11, 12, 13, 14), les deux dites paires travaillant en opposition de phase, ladite opposition de phase étant contrôlée, au moins :
∘ en faisant varier la durée de ladite translation arrière dans ledit deuxième cycle de rotation pour au moins une parmi lesdites paires,
▪ de façon à contrôler et maintenir ladite opposition de phase entre les deux dites paires.

4. Module robot de cathéter selon l'une quelconque des revendications précédentes, dans lequel :
➢ ledit pilote desdites paires de patins mobiles étant mis en œuvre de sorte que :
∘ ladite translation dudit élément médical allongé flexible étant réalisée alternativement par lesdites paires de patins mobiles, les deux dites paires travaillant en opposition de phase,
∘ ladite rotation dudit élément médical allongé flexible étant réalisée alternativement par lesdites paires de patins mobiles,
∘ un conflit de synchronisation entre ladite translation et ladite rotation étant géré, au moins :
▪ en faisant varier l'extension de course et/ou la durée de ladite translation avant, dans ledit premier cycle de translation et/ou dans ledit deuxième cycle de rotation, pour les deux dites paires,
• de façon à maintenir toujours au moins une paire de patins mobiles serrés sur ledit élément médical allongé flexible, pendant toute la durée de ladite translation dudit élément médical allongé flexible dans ledit premier cycle de translation ainsi que pendant toute la durée de ladite rotation dudit élément médical allongé flexible dans ledit deuxième cycle de rotation.

5. Module robot de cathéter selon la revendication 4, dans lequel :
➢ ledit pilote desdites paires de patins mobiles est mis en œuvre de sorte que :
∘ ladite opposition de phase est contrôlée, au moins :
▪ en faisant varier la durée de ladite translation arrière, dans ledit premier cycle de translation et dans ledit deuxième cycle de rotation, pour au moins une parmi lesdites paires,
• de façon à contrôler et maintenir ladite opposition de phase entre les deux dites paires.

6. Module robot de cathéter selon l'une quelconque des revendications précédentes, dans lequel :
➢ ledit pilote desdites paires (15, 16) de patins mobiles (11, 12, 13, 14) est mis en œuvre de sorte que :
∘ ladite translation dudit élément médical allongé flexible (10) est réalisée alternativement par lesdites paires de patins mobiles, les deux paires travaillant en opposition de phase, ladite opposition de phase étant contrôlée principalement ou uniquement :
▪ en faisant varier la durée de ladite translation arrière, dans ledit premier cycle de translation pour au moins une parmi lesdites paires,
• de façon à contrôler et maintenir ladite opposition de phase entre les deux dites paires.

7. Module robot de cathéter selon l'une quelconque des revendications précédentes, dans lequel :
➢ ledit pilote desdites paires (15, 16) de patins mobiles (11, 12, 13, 14) est mis en œuvre de sorte que :
∘ ladite rotation dudit élément médical allongé flexible (10) est alternativement réalisée par lesdites paires de patins mobiles,
∘ un conflit de synchronisation entre ladite translation et ladite rotation est géré principalement ou uniquement :
▪ en faisant varier l'extension de course de ladite translation avant, dans ledit premier cycle de translation pour au moins une parmi lesdites paires,
• de façon à maintenir toujours au moins une paire de patins mobiles serrés sur ledit élément médical allongé flexible, pendant toute la durée de ladite translation dudit élément médical allongé flexible dans ledit premier cycle ainsi que pendant toute la durée de ladite rotation dudit élément médical allongé flexible dans ledit deuxième cycle.

8. Module robot de cathéter selon l'une quelconque des revendications précédentes, dans lequel ladite durée de translation avant est toujours plus longue que ladite durée de translation arrière.

9. Module robot de cathéter selon l'une quelconque des revendications précédentes, dans lequel ladite extension de course variable de ladite translation avant dans ledit premier cycle de translation pour l'une desdites paires est réalisée en étendant une plage de course de translation avant standard prédéterminée, atteignant une valeur allant de ladite plage de course de translation avant standard prédéterminée à une plage de course de translation avant maximale prédéterminée,
et dans lequel de préférence :
ladite plage de course de translation avant maximale prédéterminée est comprise entre 110 % et 150 % de ladite plage de course de translation avant standard prédéterminée, de préférence entre 120 % et 140 % de ladite plage de course de translation avant standard prédéterminée,
et/ou dans lequel de préférence
ladite plage de course de translation avant maximale prédéterminée est divisée en deux parties égales respectivement aux deux extrémités de ladite plage de course de translation avant standard prédéterminée.

10. Module robot de cathéter selon l'une quelconque des revendications précédentes, dans lequel il existe un certain chevauchement temporaire entre ledit serrage dudit élément médical allongé flexible (10) par l'une desdites paires de patins mobiles et ledit serrage dudit élément médical allongé flexible par l'autre desdites paires de patins mobiles, ledit chevauchement temporaire durant de préférence entre 10 % et 95 % de la durée totale de ladite translation dudit élément médical allongé flexible.

11. Module de robot de cathéter selon l'une quelconque des revendications précédentes, dans lequel ledit desserrage dudit élément médical allongé flexible (10) est réalisé simultanément sur une portion de ladite extension de course de translation avant, pendant la deuxième moitié de ladite extension de course de translation avant, ladite portion allant de préférence de 5 % à 20 % de la pleine étendue de ladite extension de course de translation avant.

12. Module robot de cathéter selon l'une quelconque des revendications précédentes, dans lequel ledit serrage dudit élément médical allongé flexible (10) est réalisé simultanément sur une portion de ladite extension de course de translation avant, pendant la première moitié de ladite extension de course de translation avant, ladite portion allant de préférence de 5 % à 20 % de la pleine étendue de ladite extension de course de translation avant.

13. Module robot de cathéter selon l'une quelconque des revendications précédentes, dans lequel ledit serrage dudit élément médical allongé flexible (10) commence après la fin de ladite extension de course de translation arrière et après le début de ladite extension de course de translation avant suivante.

14. Module robot de cathéter selon l'une quelconque des revendications précédentes, dans lequel ladite variation de la durée de ladite translation arrière dans ledit premier cycle de translation pour l'une desdites paires (15, 16), de façon à contrôler et maintenir ladite opposition de phase entre les deux dites paires (15, 16), est réalisée en réduisant ou en rallongeant la durée par rapport à une durée de translation arrière standard,
et dans lequel de préférence :
ladite variation de la durée de ladite translation arrière dans ledit premier cycle de translation pour l'une desdites paires (15, 16), de façon à contrôler et maintenir ladite opposition de phase entre les deux dites paires (15, 16), est réalisée en réduisant ou en rallongeant la durée par rapport à une durée de translation arrière standard inférieure à celle demandée pour un contrôle et un maintien optimaux de l'opposition de phase de façon à améliorer la stabilité au coût d'un nombre plus élevé de cycles pour revenir à la cible d'opposition de phase, un facteur α d'atténuation de correction compris entre 0 et 1 étant appliqué,
et/ou dans lequel de préférence :
ledit facteur α d'atténuation de correction est compris entre 0,3 et 0,7, et est de préférence d'environ 0,5.

15. Module robot de cathéter selon l'une quelconque des revendications précédentes, dans lequel ladite durée de translation arrière standard est une fonction décroissante d'une (de) valeur(s) cible(s) de vitesse de commande utilisateur, pour la translation et/ou la rotation, de préférence un minimum des deux valeurs cibles de vitesse, le cas échéant, devenant une valeur cible de vitesse de commande utilisateur sélectionnée, et dans lequel de préférence :
ladite fonction décroissante présente une partie courbée centrale (280) qui présente une concavité vers le haut et qui est située entre deux parties horizontales (281, 282), et/ou dans lequel de préférence :
ladite partie courbée centrale (280) est inversement proportionnelle à ladite valeur cible de vitesse de commande utilisateur sélectionnée, tandis que lesdites parties horizontales (281, 282) sont constantes par rapport à ladite valeur cible de vitesse de commande utilisateur sélectionnée.

16. Module robot de cathéter selon l'une quelconque des revendications précédentes, dans lequel :
➢ ledit sens de translation longitudinale défini par l'utilisateur peut être varié en continu par ledit utilisateur,
➢ et/ou ledit sens de rotation défini par l'utilisateur peut être varié en continu par ledit utilisateur.

17. Module robot de cathéter selon l'une quelconque des revendications précédentes, dans lequel :
➢ ladite translation dudit élément médical allongé flexible (10) longitudinalement pour la première (16) desdites paires de patins (13, 14) est réalisée par une première machine à états finis (331),
➢ ladite rotation dudit élément médical allongé flexible autour de l'axe longitudinal par rapport audit carter pour la première (16) desdites paires de patins (13, 14) est réalisée par une deuxième machine à états finis (332),
➢ ladite translation dudit élément médical allongé flexible longitudinalement pour la deuxième (15) desdites paires de patins (11, 12) est réalisée par une troisième machine à états finis (333),
➢ ladite rotation dudit élément médical allongé flexible autour de l'axe longitudinal par rapport audit carter pour la deuxième (15) desdites paires de patins (11, 12) est réalisée par une quatrième machine à états finis (334),
et dans lequel de préférence :
➢ chacune desdites machines à états finis détermine pour une période de transition entre ladite translation avant et ladite translation arrière pour passer progressivement de ladite translation avant à ladite translation arrière :
∘ le début de ladite période de transition,
∘ la durée de ladite période de transition,
∘ la fin de ladite période de transition,
et/ou dans lequel de préférence :
chacune desdites machines à états finis change périodiquement avec une période (Δt) qui est inférieure à 5 ms, de préférence comprise entre 0,5 ms et 2 ms, de manière davantage préférée d'environ 1 ms.
